# EUROPEAN PATENT SPECIFICATION

(11) **EP 3 333 571 B1**
(45) Date of publication and mention of the grant of the patent: **30.06.2021**
(21) Application number: 16832841.7
(22) Date of filing: 26.07.2016
(51) Int. Cl.: G01N 33/68

(54) **METHOD AND KIT FOR PARALLEL QUANTIFICATION OF SPLICE VARIANTS OF VASCULAR ENDOTHELIAL GROWTH FACTOR (VEGF-A)**
VERFAHREN UND KIT ZUR GLEICHZEITIGEN QUANTIFIZIERUNG VON SPLEISSVARIANTEN DES VASKULÄREN ENDOTHELIALEN WACHSTUMSFAKTORS (VEGF-A)
PROCÉDÉ DE QUANTIFICATION PARALLÈLE DE VARIANTES D'ÉPISSAGE DU FACTEUR DE CROISSANCE ENDOTHÉLIAL VASCULAIRE (VEGF-A)

(30) Priority: 03.08.2015 JP 2015153656
(43) Date of publication of application: 13.06.2018
(73) Proprietor: Shimadzu Corporation, Kyoto-shi, Kyoto 604-8511 (JP); National Cancer Center, Tokyo 104-0045 (JP)
(72) Inventor: SHIMADA, Takashi, Kyoto-shi Kyoto 604-8511 (JP); UMINO, Yukari, Kyoto-shi Kyoto 604-8511 (JP); IWAMOTO, Noriko, Kyoto-shi Kyoto 604-8511 (JP); HAMADA, Akinobu, Tokyo 104-0045 (JP)
(74) Representative: Müller-Boré & Partner Patentanwälte PartG mbB
(86) International application number: PCT/JP2016/071821
(87) International publication number: WO 2017/022562

(56) References cited:
- WO-A2-2006/022895
- WO-A2-2009/061904
- WO-A2-2013/044265
- JP-A- 2008 529 027
- JP-A- 2009 523 440
- JP-A- 2014 238 343
- JP-A- 2015 105 951
- US-A1- 2009 140 136
- DANIEL C. LIEBLER ET AL: "Targeted Quantitation of Proteins by Mass Spectrometry", BIOCHEMISTRY, vol. 52, no. 22, 27 March 2013 (2013-03-27), pages 3797-3806, XP055550043, US ISSN: 0006-2960, DOI: 10.1021/bi400110b
- R. M. MCCULLOUGH ET AL: "High-throughput alternative splicing quantification by primer extension and matrix-assisted laser desorption/ionization time-of-flight mass spectrometry", NUCLEIC ACIDS RESEARCH, vol. 33, no. 11, 16 June 2005 (2005-06-16) , pages e99-e99, XP055550048, ISSN: 0305-1048, DOI: 10.1093/nar/gni098
- Stacy D. Sherrod ET AL: "Label-Free Quantitation of Protein Modifications by Pseudo Selected Reaction Monitoring with Internal Reference Peptides", JOURNAL OF PROTEOME RESEARCH, vol. 11, no. 6, 17 June 2012 (2012-06-17), pages 3467-3479, XP055662530, ISSN: 1535-3893, DOI: 10.1021/pr201240a

### SEQUENCE LISTING

<110> Shimadzu Corporation National Cancer Center
<120> Method of Parallel Quantification of Peptide Variants
<130> PH-6544-PCT
<150> JP 2015-153656
   <151> 2015-08-03
<160> 38
<170> PatentIn version 3.5
<210> 1
   <211> 232
   <212> PRT
   <213> Homo sapiens
<400> 1
   Met Asn Phe Leu Leu Ser Trp Val His Trp Ser Leu Ala Leu Leu Leu
   1               5                   10                  15
   Tyr Leu His His Ala Lys Trp Ser Gln Ala Ala Pro Met Ala Glu Gly
               20                  25                  30
   Gly Gly Gln Asn His His Glu Val Val Lys Phe Met Asp Val Tyr Gln
           35                  40                  45
   Arg Ser Tyr Cys His Pro Ile Glu Thr Leu Val Asp Ile Phe Gln Glu
       50                  55                  60
   Tyr Pro Asp Glu Ile Glu Tyr Ile Phe Lys Pro Ser Cys Val Pro Leu
   65                  70                  75                  80
   Met Arg Cys Gly Gly Cys Cys Asn Asp Glu Gly Leu Glu Cys Val Pro
                   85                  90                  95
   Thr Glu Glu Ser Asn Ile Thr Met Gln Ile Met Arg Ile Lys Pro His
               100                 105                 110
   Gln Gly Gln His Ile Gly Glu Met Ser Phe Leu Gln His Asn Lys Cys
           115                 120                 125
   Glu Cys Arg Pro Lys Lys Asp Arg Ala Arg Gln Glu Lys Lys Ser Val
       130                 135                 140
   Arg Gly Lys Gly Lys Gly Gln Lys Arg Lys Arg Lys Lys Ser Arg Tyr
   145                 150                 155                 160
   Lys Ser Trp Ser Val Tyr Val Gly Ala Arg Cys Cys Leu Met Pro Trp
                   165                 170                 175
   Ser Leu Pro Gly Pro His Pro Cys Gly Pro Cys Ser Glu Arg Arg Lys
               180                 185                 190
   His Leu Phe Val Gln Asp Pro Gln Thr Cys Lys Cys Ser Cys Lys Asn
           195                 200                 205
   Thr Asp Ser Arg Cys Lys Ala Arg Gln Leu Glu Leu Asn Glu Arg Thr
       210                 215                 220
   Cys Arg Cys Asp Lys Pro Arg Arg
   225                 230
<210> 2
   <211> 215
   <212> PRT
   <213> Homo sapiens
<400> 2
   Met Asn Phe Leu Leu Ser Trp Val His Trp Ser Leu Ala Leu Leu Leu
   1               5                   10                  15
   Tyr Leu His His Ala Lys Trp Ser Gln Ala Ala Pro Met Ala Glu Gly
               20                  25                  30
   Gly Gly Gln Asn His His Glu Val Val Lys Phe Met Asp Val Tyr Gln
           35                  40                  45
   Arg Ser Tyr Cys His Pro Ile Glu Thr Leu Val Asp Ile Phe Gln Glu
       50                  55                  60
   Tyr Pro Asp Glu Ile Glu Tyr Ile Phe Lys Pro Ser Cys Val Pro Leu
   65                  70                  75                  80
   Met Arg Cys Gly Gly Cys Cys Asn Asp Glu Gly Leu Glu Cys Val Pro
                   85                  90                  95
   Thr Glu Glu Ser Asn Ile Thr Met Gln Ile Met Arg Ile Lys Pro His
               100                 105                 110
   Gln Gly Gln His Ile Gly Glu Met Ser Phe Leu Gln His Asn Lys Cys
           115                 120                 125
   Glu Cys Arg Pro Lys Lys Asp Arg Ala Arg Gln Glu Lys Lys Ser Val
       130                 135                 140
   Arg Gly Lys Gly Lys Gly Gln Lys Arg Lys Arg Lys Lys Ser Arg Tyr
   145 150 155 160
   Lys Ser Trp Ser Val Pro Cys Gly Pro Cys Ser Glu Arg Arg Lys His
   165 170 175
   Leu Phe Val Gln Asp Pro Gln Thr Cys Lys Cys Ser Cys Lys Asn Thr
   180 185 190
   Asp Ser Arg Cys Lys Ala Arg Gln Leu Glu Leu Asn Glu Arg Thr Cys
   195 200 205
   Arg Cys Asp Lys Pro Arg Arg
   210 215
<210> 3
   <211> 209
   <212> PRT
   <213> Homo sapiens
<400> 3
   Met Asn Phe Leu Leu Ser Trp Val His Trp Ser Leu Ala Leu Leu Leu
   1 5 10 15
   Tyr Leu His His Ala Lys Trp Ser Gln Ala Ala Pro Met Ala Glu Gly
   20 25 30
   Gly Gly Gln Asn His His Glu Val Val Lys Phe Met Asp Val Tyr Gln
   35 40 45
   Arg Ser Tyr Cys His Pro Ile Glu Thr Leu Val Asp Ile Phe Gln Glu
   50 55 60
   Tyr Pro Asp Glu Ile Glu Tyr Ile Phe Lys Pro Ser Cys Val Pro Leu
   65 70 75 80
   Met Arg Cys Gly Gly Cys Cys Asn Asp Glu Gly Leu Glu Cys Val Pro
   85 90 95
   Thr Glu Glu Ser Asn Ile Thr Met Gln Ile Met Arg Ile Lys Pro His
   100 105 110
   Gln Gly Gln His Ile Gly Glu Met Ser Phe Leu Gln His Asn Lys Cys
   115 120 125
   Glu Cys Arg Pro Lys Lys Asp Arg Ala Arg Gln Glu Lys Lys Ser Val
   130 135 140
   Arg Gly Lys Gly Lys Gly Gln Lys Arg Lys Arg Lys Lys Ser Arg Pro
   145 150 155 160
   Cys Gly Pro Cys Ser Glu Arg Arg Lys His Leu Phe Val Gln Asp Pro
   165 170 175
   Gln Thr Cys Lys Cys Ser Cys Lys Asn Thr Asp Ser Arg Cys Lys Ala
   180 185 190
   Arg Gln Leu Glu Leu Asn Glu Arg Thr Cys Arg Cys Asp Lys Pro Arg
   195 200 205
   Arg
<210> 4
   <211> 191
   <212> PRT
   <213> Homo sapiens
<400> 4
   Met Asn Phe Leu Leu Ser Trp Val His Trp Ser Leu Ala Leu Leu Leu
   1 5 10 15
   Tyr Leu His His Ala Lys Trp Ser Gln Ala Ala Pro Met Ala Glu Gly
   20 25 30
   Gly Gly Gln Asn His His Glu Val Val Lys Phe Met Asp Val Tyr Gln
   35 40 45
   Arg Ser Tyr Cys His Pro Ile Glu Thr Leu Val Asp Ile Phe Gln Glu
   50 55 60
   Tyr Pro Asp Glu Ile Glu Tyr Ile Phe Lys Pro Ser Cys Val Pro Leu
   65 70 75 80
   Met Arg Cys Gly Gly Cys Cys Asn Asp Glu Gly Leu Glu Cys Val Pro
   85 90 95
   Thr Glu Glu Ser Asn Ile Thr Met Gln Ile Met Arg Ile Lys Pro His
   100 105 110
   Gln Gly Gln His Ile Gly Glu Met Ser Phe Leu Gln His Asn Lys Cys
   115 120 125
   Glu Cys Arg Pro Lys Lys Asp Arg Ala Arg Gln Glu Asn Pro Cys Gly
   130 135 140
   Pro Cys Ser Glu Arg Arg Lys His Leu Phe Val Gln Asp Pro Gln Thr
   145                 150                 155                 160
   Cys Lys Cys Ser Cys Lys Asn Thr Asp Ser Arg Cys Lys Ala Arg Gln
                   165                 170                 175
   Leu Glu Leu Asn Glu Arg Thr Cys Arg Cys Asp Lys Pro Arg Arg
               180                 185                 190
<210> 5
   <211> 174
   <212> PRT
   <213> Homo sapiens
<400> 5
   Met Asn Phe Leu Leu Ser Trp Val His Trp Ser Leu Ala Leu Leu Leu
   1               5                   10                  15
   Tyr Leu His His Ala Lys Trp Ser Gln Ala Ala Pro Met Ala Glu Gly
               20                  25                  30
   Gly Gly Gln Asn His His Glu Val Val Lys Phe Met Asp Val Tyr Gln
           35                  40                  45
   Arg Ser Tyr Cys His Pro Ile Glu Thr Leu Val Asp Ile Phe Gln Glu
       50                  55                  60
   Tyr Pro Asp Glu Ile Glu Tyr Ile Phe Lys Pro Ser Cys Val Pro Leu
   65                  70                  75                  80
   Met Arg Cys Gly Gly Cys Cys Asn Asp Glu Gly Leu Glu Cys Val Pro
                   85                  90                  95
   Thr Glu Glu Ser Asn Ile Thr Met Gln Ile Met Arg Ile Lys Pro His
               100                 105                 110
   Gln Gly Gln His Ile Gly Glu Met Ser Phe Leu Gln His Asn Lys Cys
           115                 120                 125
   Glu Cys Arg Pro Lys Lys Asp Arg Ala Arg Gln Glu Asn Pro Cys Gly
       130                 135                 140
   Pro Cys Ser Glu Arg Arg Lys His Leu Phe Val Gln Asp Pro Gln Thr
   145                 150                 155                 160
   Cys Lys Cys Ser Cys Lys Asn Thr Asp Ser Arg Cys Lys Met
                   165                 170
<210> 6
   <211> 171
   <212> PRT
   <213> Homo sapiens
<400> 6
   Met Asn Phe Leu Leu Ser Trp Val His Trp Ser Leu Ala Leu Leu Leu
   1               5                   10                  15
   Tyr Leu His His Ala Lys Trp Ser Gln Ala Ala Pro Met Ala Glu Gly
               20                  25                  30
   Gly Gly Gln Asn His His Glu Val Val Lys Phe Met Asp Val Tyr Gln
           35                  40                  45
   Arg Ser Tyr Cys His Pro Ile Glu Thr Leu Val Asp Ile Phe Gln Glu
       50                  55                  60
   Tyr Pro Asp Glu Ile Glu Tyr Ile Phe Lys Pro Ser Cys Val Pro Leu
   65                  70                  75                  80
   Met Arg Cys Gly Gly Cys Cys Asn Asp Glu Gly Leu Glu Cys Val Pro
                   85                  90                  95
   Thr Glu Glu Ser Asn Ile Thr Met Gln Ile Met Arg Ile Lys Pro His
               100                 105                 110
   Gln Gly Gln His Ile Gly Glu Met Ser Phe Leu Gln His Asn Lys Cys
           115                 120                 125
   Glu Cys Arg Pro Lys Lys Asp Arg Ala Arg Gln Glu Lys Lys Ser Val
       130                 135                 140
   Arg Gly Lys Gly Lys Gly Gln Lys Arg Lys Arg Lys Lys Ser Arg Tyr
   145                 150                 155                 160
   Lys Ser Trp Ser Val Cys Asp Lys Pro Arg Arg
                   165                 170
<210> 7
   <211> 147
   <212> PRT
   <213> Homo sapiens
<400> 7
   Met Asn Phe Leu Leu Ser Trp Val His Trp Ser Leu Ala Leu Leu Leu
   1               5                   10                  15
   Tyr Leu His His Ala Lys Trp Ser Gln Ala Ala Pro Met Ala Glu Gly
                   20                  25                  30
   Gly Gly Gln Asn His His Glu Val Val Lys Phe Met Asp Val Tyr Gln
           35                  40                  45
   Arg Ser Tyr Cys His Pro Ile Glu Thr Leu Val Asp Ile Phe Gln Glu
       50                  55                  60
   Tyr Pro Asp Glu Ile Glu Tyr Ile Phe Lys Pro Ser Cys Val Pro Leu
   65                  70                  75                  80
   Met Arg Cys Gly Gly Cys Cys Asn Asp Glu Gly Leu Glu Cys Val Pro
                   85                  90                  95
   Thr Glu Glu Ser Asn Ile Thr Met Gln Ile Met Arg Ile Lys Pro His
               100                 105                 110
   Gln Gly Gln His Ile Gly Glu Met Ser Phe Leu Gln His Asn Lys Cys
           115                 120                 125
   Glu Cys Arg Pro Lys Lys Asp Arg Ala Arg Gln Glu Asn Cys Asp Lys
       130                 135                 140
   Pro Arg Arg
   145
<210> 8
   <211> 191
   <212> PRT
   <213> Homo sapiens
<400> 8
   Met Asn Phe Leu Leu Ser Trp Val His Trp Ser Leu Ala Leu Leu Leu
   1               5                   10                  15
   Tyr Leu His His Ala Lys Trp Ser Gln Ala Ala Pro Met Ala Glu Gly
               20                  25                  30
   Gly Gly Gln Asn His His Glu Val Val Lys Phe Met Asp Val Tyr Gln
           35                  40                  45
   Arg Ser Tyr Cys His Pro Ile Glu Thr Leu Val Asp Ile Phe Gln Glu
       50                  55                  60
   Tyr Pro Asp Glu Ile Glu Tyr Ile Phe Lys Pro Ser Cys Val Pro Leu
   65                  70                  75                  80
   Met Arg Cys Gly Gly Cys Cys Asn Asp Glu Gly Leu Glu Cys Val Pro
                   85                  90                  95
   Thr Glu Glu Ser Asn Ile Thr Met Gln Ile Met Arg Ile Lys Pro His
               100                 105                 110
   Gln Gly Gln His Ile Gly Glu Met Ser Phe Leu Gln His Asn Lys Cys
           115                 120                 125
   Glu Cys Arg Pro Lys Lys Asp Arg Ala Arg Gln Glu Asn Pro Cys Gly
       130                 135                 140
   Pro Cys Ser Glu Arg Arg Lys His Leu Phe Val Gln Asp Pro Gln Thr
   145                 150                 155                 160
   Cys Lys Cys Ser Cys Lys Asn Thr Asp Ser Arg Cys Lys Ala Arg Gln
                   165                 170                 175
   Leu Glu Leu Asn Glu Arg Thr Cys Arg Ser Leu Thr Arg Lys Asp
               180                 185                 190
<210> 9
   <211> 147
   <212> PRT
   <213> Homo sapiens
<400> 9
   Met Asn Phe Leu Leu Ser Trp Val His Trp Ser Leu Ala Leu Leu Leu
   1               5                   10                  15
   Tyr Leu His His Ala Lys Trp Ser Gln Ala Ala Pro Met Ala Glu Gly
               20                  25                  30
   Gly Gly Gln Asn His His Glu Val Val Lys Phe Met Asp Val Tyr Gln
           35                  40                  45
   Arg Ser Tyr Cys His Pro Ile Glu Thr Leu Val Asp Ile Phe Gln Glu
       50                  55                  60
   Tyr Pro Asp Glu Ile Glu Tyr Ile Phe Lys Pro Ser Cys Val Pro Leu
   65                  70                  75                  80
   Met Arg Cys Gly Gly Cys Cys Asn Asp Glu Gly Leu Glu Cys Val Pro
                   85                  90                  95
   Thr Glu Glu Ser Asn Ile Thr Met Gln Ile Met Arg Ile Lys Pro His
               100                 105                 110
   Gln Gly Gln His Ile Gly Glu Met Ser Phe Leu Gln His Asn Lys Cys
           115                 120                 125
   Glu Cys Arg Pro Lys Lys Asp Arg Ala Arg Gln Glu Lys Cys Asp Lys
       130                 135                 140
   Pro Arg Arg
   145
<210> 10
   <211> 137
   <212> PRT
   <213> Homo sapiens
<400> 10
   Met Asn Phe Leu Leu Ser Trp Val His Trp Ser Leu Ala Leu Leu Leu
   1               5                   10                  15
   Tyr Leu His His Ala Lys Trp Ser Gln Ala Ala Pro Met Ala Glu Gly
               20                  25                  30
   Gly Gly Gln Asn His His Glu Val Val Lys Phe Met Asp Val Tyr Gln
           35                  40                  45
   Arg Ser Tyr Cys His Pro Ile Glu Thr Leu Val Asp Ile Phe Gln Glu
       50                  55                  60
   Tyr Pro Asp Glu Ile Glu Tyr Ile Phe Lys Pro Ser Cys Val Pro Leu
   65                  70                  75                  80
   Met Arg Cys Gly Gly Cys Cys Asn Asp Glu Gly Leu Glu Cys Val Pro
                   85                  90                  95
   Thr Glu Glu Ser Asn Ile Thr Met Gln Ile Met Arg Ile Lys Pro His
               100                 105                 110
   Gln Gly Gln His Ile Gly Glu Met Ser Phe Leu Gln His Asn Lys Cys
           115                 120                 125
   Glu Cys Arg Cys Asp Lys Pro Arg Arg
       130                 135
<210> 11
   <211> 16
   <212> PRT
   <213> Homo sapiens
<400> 11
   Ala Pro Met Ala Glu Gly Gly Gly Gln Asn His His Glu Val Val Lys
   1               5                   10                  15
<210> 12
   <211> 7
   <212> PRT
   <213> Homo sapiens
<400> 12
   Phe Met Asp Val Tyr Gln Arg
   1               5
<210> 13
   <211> 19
   <212> PRT
   <213> Homo sapiens
<400> 13
   Ile Lys Pro His Gln Gly Gln His Ile Gly Glu Met Ser Phe Leu Gln
   1 5 10 15
   His Asn Lys
<210> 14
   <211> 6
   <212> PRT
   <213> Homo sapiens
<400> 14
   Cys Glu Cys Arg Pro Lys
   1 5
<210> 15
   <211> 9
   <212> PRT
   <213> Homo sapiens
<400> 15
   Ser Trp Ser Val Tyr Val Gly Ala Arg
   1 5
<210> 16
   <211> 20
   <212> PRT
   <213> Homo sapiens
<400> 16
   Cys Cys Leu Met Pro Trp Ser Leu Pro Gly Pro His Pro Cys Gly Pro
   1 5 10 15
   Cys Ser Glu Arg
   20
<210> 17
   <211> 11
   <212> PRT
   <213> Homo sapiens
<400> 17
   His Leu Phe Val Gln Asp Pro Gln Thr Cys Lys
   1 5 10
<210> 18
   <211> 5
   <212> PRT
   <213> Homo sapiens
<400> 18
   Asn Thr Asp Ser Arg
   1 5
<210> 19
   <211> 7
   <212> PRT
   <213> Homo sapiens
<400> 19
   Gln Leu Glu Leu Asn Glu Arg
   1 5
<210> 20
   <211> 5
   <212> PRT
   <213> Homo sapiens
<400> 20
   Cys Asp Lys Pro Arg
   1 5
<210> 21
   <211> 12
   <212> PRT
   <213> Homo sapiens
<400> 21
   Ser Trp Ser Val Pro Cys Gly Pro Cys Ser Glu Arg
   1 5 10
<210> 22
   <211> 10
   <212> PRT
   <213> Homo sapiens
<400> 22
   Ser Arg Pro Cys Gly Pro Cys Ser Glu Arg
   1 5 10
<210> 23
   <211> 11
   <212> PRT
   <213> Homo sapiens
<400> 23
   Gln Glu Asn Pro Cys Gly Pro Cys Ser Glu Arg
   1 5 10
<210> 24
   <211> 9
   <212> PRT
   <213> Homo sapiens
<400> 24
   Ser Trp Ser Val Cys Asp Lys Pro Arg
   1 5
<210> 25
   <211> 8
   <212> PRT
   <213> Homo sapiens
<400> 25
   Gln Glu Asn Cys Asp Lys Pro Arg
   1 5
<210> 26
   <211> 5
   <212> PRT
   <213> Homo sapiens
<400> 26
   Ser Leu Thr Arg Lys
   1 5
<210> 27
   <211> 14
   <212> PRT
   <213> Homo sapiens
<400> 27
   Met Ala Glu Gly Gly Gly Gln Asn His His Glu Val Val Lys
   1 5 10
<210> 28
   <211> 5
   <212> PRT
   <213> Homo sapiens
<400> 28
   Asp Val Tyr Gln Arg
   1 5
<210> 29
   <211> 17
   <212> PRT
   <213> Homo sapiens
<400> 29
   Pro His Gln Gly Gln His Ile Gly Glu Met Ser Phe Leu Gln His Asn
   1 5 10 15
   Lys
<210> 30
   <211> 4
   <212> PRT
   <213> Homo sapiens
<400> 30
   Cys Arg Pro Lys
   1
<210> 31
   <211> 7
   <212> PRT
   <213> Homo sapiens
<400> 31
   Ser Val Tyr Val Gly Ala Arg
   1 5
<210> 32
   <211> 15
   <212> PRT
   <213> Homo sapiens
<400> 32
   Pro Trp Ser Leu Pro Gly Pro His Pro Cys Gly Pro Cys Ser Glu
   1 5 10 15
<210> 33
   <211> 5
   <212> PRT
   <213> Homo sapiens
<400> 33
   Pro Gln Thr Cys Lys
   1 5
<210> 34
   <211> 5
   <212> PRT
   <213> Homo sapiens
<400> 34
   Glu Leu Asn Glu Arg
   1 5
<210> 35
   <211> 8
   <212> PRT
   <213> Homo sapiens
<400> 35
   Pro Cys Gly Pro Cys Ser Glu Arg
   1 5
<210> 36
   <211> 4
   <212> PRT
   <213> Homo sapiens
<400> 36
   Cys Ser Glu Arg
   1
<210> 37
   <211> 8
   <212> PRT
   <213> Homo sapiens
<400> 37
   Pro Cys Gly Pro Cys Ser Glu Arg
   1 5
<210> 38
   <211> 5
   <212> PRT
   <213> Homo sapiens
<400> 38
   Cys Asp Lys Pro Arg
   1 5

## Description

The present invention relates to a method for parallel quantification of protein variants, more specifically, relates to a method that allows protein variants that can be mixed in a sample to be simultaneously detected and quantified using mass spectrometry without separating the protein variants, wherein the protein is a vascular endothelial growth factor (VEGF)-A.

Similar to a normal tissue, supply of nutrients is essential also in a proliferative process of cancer, and at the same time, excretion of wastes generated in a proliferative process is also important. Therefore, as cancer proliferates, it draws new blood vessels into its own tissues and tries to survive. In order to draw in blood vessels, it is necessary to promote growth of blood vessels. Therefore, cancer tissues produce a blood vessel growth factor and draw in new blood vessels. In this way, by securing supply of nutrients and a waste excretion route, it is possible for cancer tissues to further proliferate. As such a growth factor, a vascular endothelial growth factor (VEGF) is a typical factor.

In cancer chemotherapy, a strategy has been adopted to attack supply of cancer by inhibiting an effect of this growth factor and suppressing generation of new blood vessels. For example, bevacizumab (trade name: Avastin), which is a monoclonal antibody, is an antibody drug having an anti-cancer effect achieved by neutralizing a VEGF-A that belongs to a VEGF family. By binding to a binding site of a receptor of a VEGF, bevacizumab competitively inhibits binding of the VEGF to the receptor and inhibits activation of the receptor by the VEGF. Further, as molecularly targeted drugs of low molecular weight compounds, sorafenib, sunitinib and the like are known that inhibit tyrosine kinase activity of a VEGF receptor.

VEGF-A is a growth factor discovered in the 1980's, and it is now known that various splicing variants exist from combinations of exons (Non-Patent Documents 1 - 4). A large number of splicing variants are produced due to differences in combinations of 8 exons and in 3'- region splicing sites. A variant spliced at a splicing site proximal to an eighth site exon (proximal splicing site (PSS)) is expressed as VEGF-Axxx, and a variant spliced at a splicing site distal to the eighth site exon (distal splicing site (DSS)) is expressed as VEGF-Axxxb. It is believed that there are mainly variants called VEGF-A206, VEGF-A189, VEGF-A183, VEGF-A165, VEGF-A148, VEGF-A145, VEGF-A121, VEGF-A189b, VEGF-A183b, VEGF-A165b, VEGF-A145b, and VEGF-A121b. Among these variants, the VEGF-Axxx type variants have an angiogenesis promoting effect, while the VEGF-Axxxb type variants conversely have an angiogenesis inhibiting effect. By maintaining an exquisite balance among these variant expressions, vascular proliferation of a living body and production of new blood vessels are controlled.

Although about 70% of variant production by alternative splicing is determined by gene expression control, it has been reported that splicing is controlled as disease such as cancer progresses. That is, there are variant expression control associated with an angiogenesis promoting effect and a variant expression mechanism due to molecular switch associated cell malignancy.

A most common technique for quantification of a protein is ELISA (Enzyme-Linked ImmunoSorbent Assay). This is a technique for easily quantifying molecules to be measured by preparing an antibody with respect to a protein to be measured and further sandwiching with detection antibodies. As a solid-phase ELISA kit for quantifying VEGF-A, for example, a Human VEGF Quantikine ELISA Kit (http://www.rndsystems.com/Products/DVE00) of R&D Systems Inc. can be used. According to this, measurement ranges are 15.6 - 1,000 pg/mL (cell culture supernatant) and 31.2 - 2,000 pg/mL (serum or plasma). Although many other ELISA kits are commercially available (IBL, Funakoshi, Sigma, Pierce, Life Technologies, and the like), these are kits that quantify a total VEGF-A amount or kits that can quantify some variants. A multiplex ELISA called a 3-ELISA system that simultaneously measures VEGF-A 165, VEGF-A 121 and VEGF-A 110 has also been developed (Non-Patent Document 5).

Further, in addition to VEGF-A, various proteins are known for which splicing variants exist. Further, for example, although Human B-type natriuretic peptide (BNP) is a peptide formed of 32 amino acids, it is known that a processed variant is present in blood and a ratio of the mixed variant can be a biomarker after a treatment of an ischemic heart disease (Non-Patent Document 6).
[Non-Patent Document 1] Harper, S. J. et al., VEGF-A splicing: the key to anti-angiogenic therapeutics?, Nat Rev Cancer, 2008
[Non-Patent Document 2] Nowak, D. G. et al., Expression of pro- and anti-angiogenic isoforms of VEGF is differentially regulated by splicing and growth factors, J Cell Sci, 2008
[Non-Patent Document 3] Rennel, E. S. et al., VEGF121b, a new member of the VEGF (xxx) b family of VEGF-A splice isoforms, inhibits neovascularisation and tumour growth in vivo, Br J Cancer, 2009
[Non-Patent Document 4] Woolard, J. et al., VEGF165b, an inhibitory vascular endothelial growth factor splice variant: mechanism of action, in vivo effect on angiogenesis and endogenous protein expression, Cancer Res, 2004
[Non-Patent Document 5] Gutierrez, J. et al., A new ELISA for use in a 3-ELISA system to assess concentrations of VEGF splice variants and VEGF (110) in ovarian cancer tumors, Clin Chem, 2008
[Non-Patent Document 6] Fujimoto, H. et al., Processed B-Type Natriuretic Peptide Is a Biomarker of Postinterventional Restenosis in Ischemic Heart Disease

In this context, WO 2009/061904 A2 describes a mass spectrometry-based quantitative assay for 4E/4E regulon pathway proteins. Further, WO 2006/082389 A1 describes a method for screening for variant peptides using mass spectrometry (MS), as well as a system and a kit for performing said method. Further, WO 2013/044265 A2 describes specific peptides and derived ionization characteristics of peptides from estrogen receptor (ER), progesterone receptor (PR), and/or antigen Ki67 (Ki67) proteins that can be used for quantifying said proteins directly in biological samples that have been fixed in formalin. Further, Liebler and Zimmermann (Liebler, D. C. and Zimmerman, L. J.; Biochemistry, 52(22); 2013; pp. 3797-3806) describes means for the targeted quantitation of proteins by mass spectrometry. Further, US 2009/0140136 A1 describes means for the detailed analysis of the primary structure or the modification state of a protein. Furthermore, McCullough *et al.* (McCullough, R. M. et al.; Nucleic Acids Res., 33(11); 2005; pp. e99) describes a method for the quantification of alternative splicing variants using primer extension and MALDI-TOF mass spectrometry. Moreover, WO 2006/022895 A2 describes methods, devices and kits for the detection of clinical conditions having associated changes in systemic angiogenic activity. Finally, Sherrod *et al.* (Sherrod, S. D. et al.; J Proteome Res., 11(6); 2012; pp. 3467-3479) describes the label-free quantitation of protein modifications by pseudo selected reaction monitoring with internal reference peptides.

ELISA does not directly measure a target to be measured. Therefore, there are many problems such as that an abnormal value may be generated, that it takes time and cost since it is necessary to produce an antibody for each target to be measured, and that it is impossible to simultaneously measure multiple analysis targets.

Further, for example, VEGF-A is a protein having a complex and characteristic structure called a cysteine knot protein. Therefore, although it is easy to prepare antibodies of VEGF-A and there are a large number of antibodies of VEGF-A, it is extremely difficult to prepare high-affinity antibodies specific to variants of the antibodies of VEGF-A. Therefore, even for an antibody capable of quantifying a variant, it is assumed that nonspecific binding cannot be sufficiently suppressed and variation in data increases.

Analysis conditions of ELISA adopted in an animal testing phase are often not directly applicable to large animals or humans because of interspecific crossing problems or the like. Therefore, in a drug discovery development stage and in a human clinical trial, it is inevitable to perform comparison under separate measurement conditions. Further, in ELISA of a drug concentration in a lesion tissue, matrix components that inhibit detection are different. Therefore, in order to perform pharmacokinetics based on ELISA, it is necessary to prepare multiple antibodies. This has a very large risk such as enormous cost in drug development and dropout in late-stage development.

Further, since a monoclonal antibody is a very expensive medicine, its use should be limited to a case where a medicinal effect is expected, and it is desirable that its medicinal effect can be predicted prior to administration to a patient. However, as described above, the anti-cancer effect of bevacizumab is achieved by binding to a VEGF-A and inhibits angiogenesis by neutralizing activity of the VEGF-A. However, this VEGF-A binding site is equally possessed by variants having a promoting effect and an inhibiting effect with respect to angiogenesis. Therefore, measuring a total amount of VEGF-A in a sample derived from a patient to be treated does not accurately reflect an anticipated medicinal effect. This is the same for other proteins that have different in vivo effects depending on variants.

For the reasons described above, in order to gain a more accurate understanding of an in vivo effect of a protein, when the protein in a sample is quantified, it is necessary to measure an amount of each variant that can be present.

Quantitative and structural analysis of a protein using mass spectrometry has been dramatically expanded in its application range, along with developments of mass spectrometry technologies and data analysis server and software. In particular, an absolute quantification technique using mass spectrometry has increased awareness as a method independent of specific antibodies. The present inventors examined possibility of simultaneously quantifying variants of a protein using mass spectrometry.

As an example, in order to be able to quantify all VEGF-A variants at once, a peptide fragment obtained by protease digestion from each variant was designed. Then, by selecting a peptide having an amino acid sequence specific to each variant, it was possible to develop an analytical condition that enables an all-at-once quantitative analysis of the variants by detecting them in parallel using mass spectrometry. The method developed by the present inventors is not only applicable to VEGF-A, but is also similarly applicable to a protein having 2 or more variants.

That is, the present invention includes the following aspects.
1. A method for parallel determination of multiple splicing variants produced by alternative splicing of a protein having 2 or more variants using mass spectrometry, comprising:
   designing peptides obtained by protease digestion of all variants of the protein in a sample;
   protease-digesting the protein;
   detecting, using mass spectrometry, among the peptides designed, 2 or more kinds of peptides having an amino acid sequence specific to each of the splicing variants and a peptide having an amino acid sequence common to 2 or more splicing variants among the multiple splicing variants; and
   determining an amount of each of the splicing variants in the sample based on a result of the mass spectrometry,
   wherein the protein is a vascular endothelial growth factor (VEGF)-A.
2. The method according to item 1, wherein the splicing variants include 2 or more selected from the following splicing variants of VEGF-A: 206 (SEQ ID NO: 1), 189 (SEQ ID NO: 2), 183 (SEQ ID NO: 3), 165 (SEQ ID NO: 4), 148 (SEQ ID NO: 5), 145 (SEQ ID NO: 6), 121 (SEQ ID NO: 7), 165b (SEQ ID NO: 8), 121b (SEQ ID NO: 9), and 111 (SEQ ID NO: 10).
3. The method according to item 1 or 2, wherein 1 or more peptides having amino acid sequences shown in SEQ ID NOs: 11 to 26 are detected.
4. A kit for detecting vascular endothelial growth factor (VEGF-A) by executing the method according to any one of items 1 to 3 using high performance liquid chromatograph mass spectrometry (LC-MS) comprising:
   a protease;
   a reaction container for digesting the protein by bringing the protein and the protease into contact with each other;
   a buffer solution for causing a digestion reaction due to the protease to occur; and
   a set of peptides as internal standard peptides having the amino acid sequences shown in SEQ ID NOs: 11 to 26.
5. The kit according to item 4 further comprising an instruction manual describing a mass spectrometry condition for detecting the splicing variants.

According to the present invention, it is possible to detect, all at once, amounts of variants that are mixed in a sample and have different effects, and it is possible to collectively acquire information that is only partially obtained in the case of using the conventional ELISA.

For example, all-at-once quantification of VEGF-A variants becomes possible by setting an optimization condition in mass spectrometry quantification of VEGF-A. By accurately detecting and quantifying each variant, it is possible to gain an understanding of to what extent each individual cancer patient has been exposed to an angiogenesis promoting effect or an angiogenesis inhibiting effect. By regulating the proliferation of cancer, it is possible to establish a more effective dosing strategy. That is, in a case of a highly proliferative cancer, chemotherapy based on neutralization with an antibody can be a first priority and can be a medication method that allows a maximum medicinal efficacy to be obtained. On the other hand, in a case where it is judged that proliferation is not so high, it is preferable to prioritize a low molecular anticancer drug and radiotherapy. Therefore, it is possible to provide an anti-cancer treatment according to each individual patient.

For a functional protein such as a growth factor and cytokine, there are a large number of variants having different functions. For example, even in immunomonitoring in order to know a medicinal effect, it is possible to formulate a current medicinal effect indicator by quantifying multiple cytokine variants all at once.

Further, also in a basic research field, it becomes possible to see an activity indicator of each variant by performing a correlation analysis between values of all-at-once quantification of hormonal molecule variants (such as growth factors) and biological functions.

The figures show:

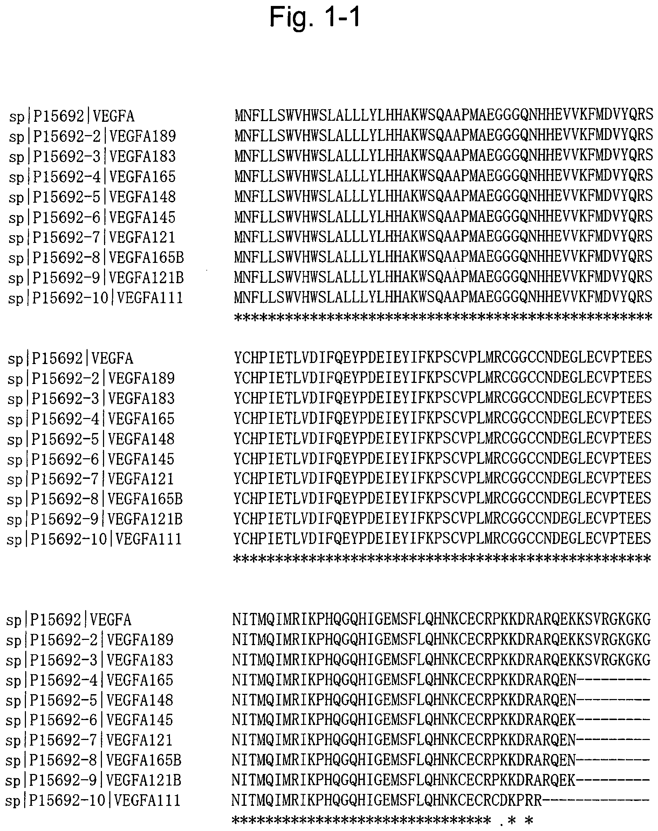

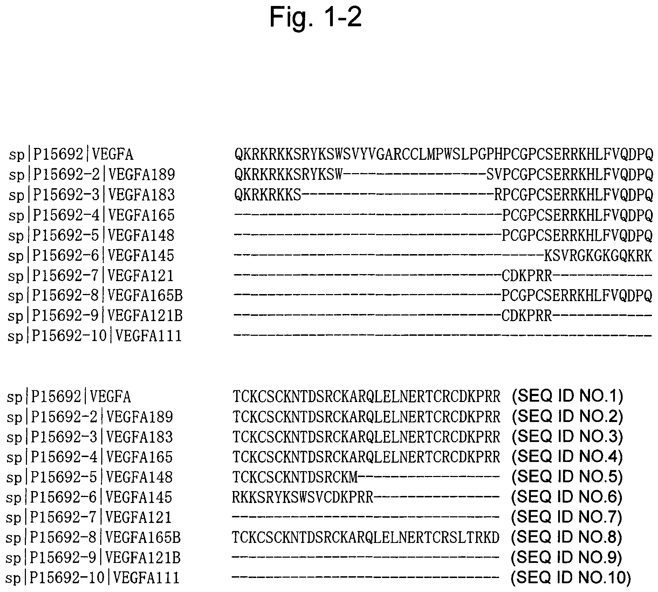

[Fig. 1-1] Fig. 1-1 shows sequence alignment of VEGFA variants.
[Fig. 1-2] Fig. 1-2 is continuation of Fig. 1-1.
[Fig. 2-1] Fig. 2-1 shows results of a preliminary MS analysis and a Q3 scan analysis of a peptide P09 (SEQ ID NO: 19).
[Fig. 2-2] Fig. 2-2 shows MS2 analysis results of the peptide P09 (SEQ ID NO: 19) and peaks of expected product ions obtained by an analysis of a selected precursor ion and detected product ions.

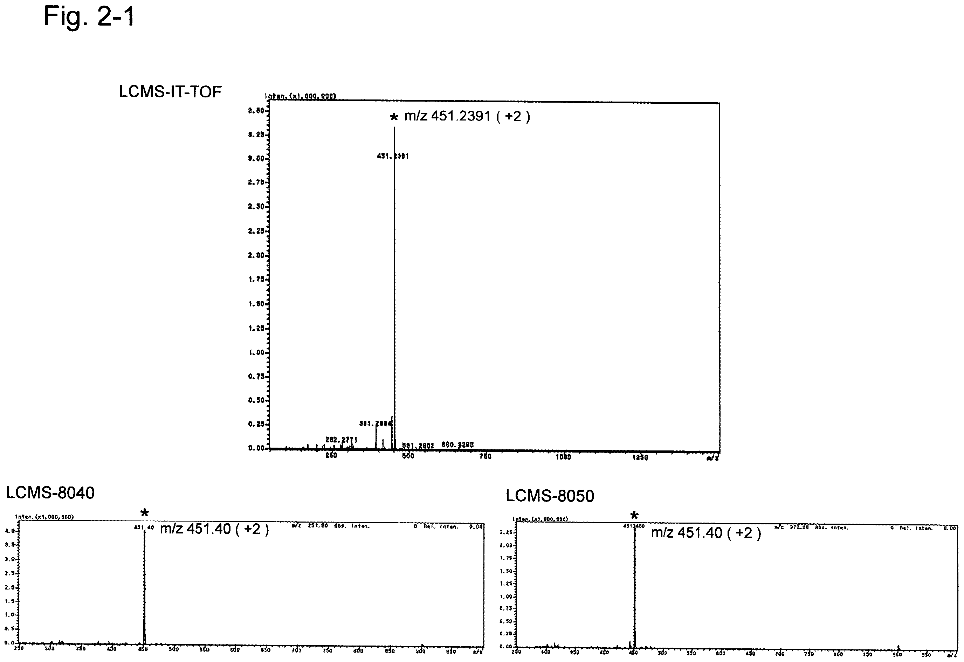

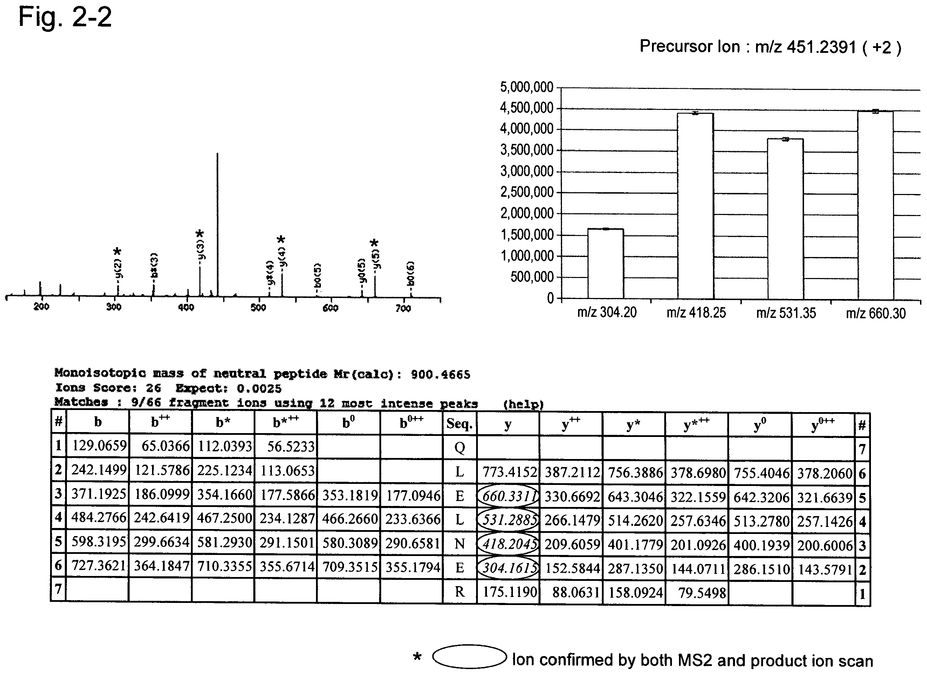

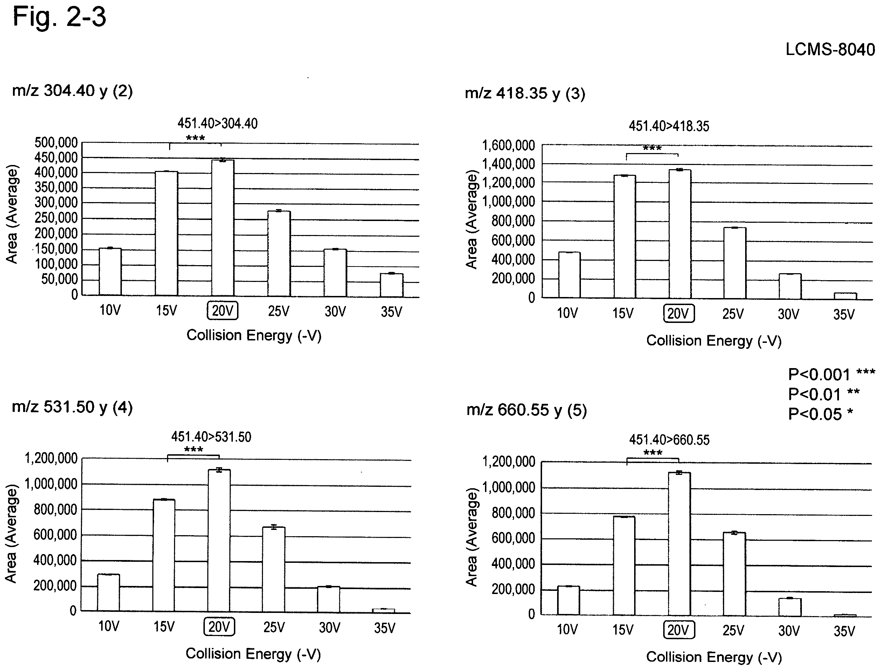

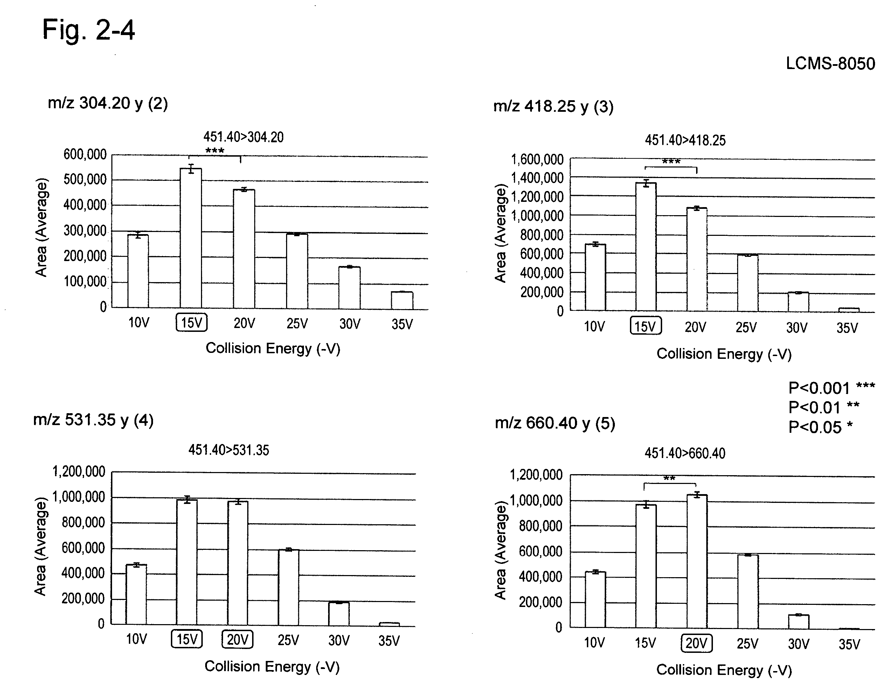

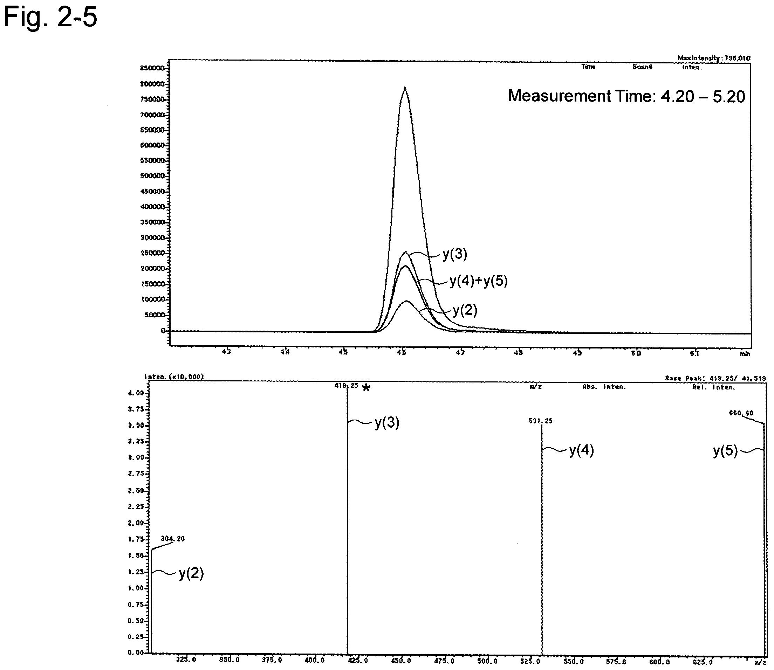

[Fig. 2-3] Fig. 2-3 shows examination results of optimization conditions of collision energies for detecting product ions to be used for analyzing the peptide P09 (SEQ ID NO: 19) (in a case where LCMS-8040 was used).
[Fig. 2-4] Fig. 2-4 shows examination results of optimization conditions of collision energies for detecting product ions to be used for analyzing the peptide P09 (SEQ ID NO: 19) (in a case where LCMS-8050 was used).
[Fig. 2-5] Fig. 2-5 shows results of quantifying the peptide P09 (SEQ ID NO: 19) by detecting y(2), y(3), y(4) and y(5) ions under the optimization conditions.

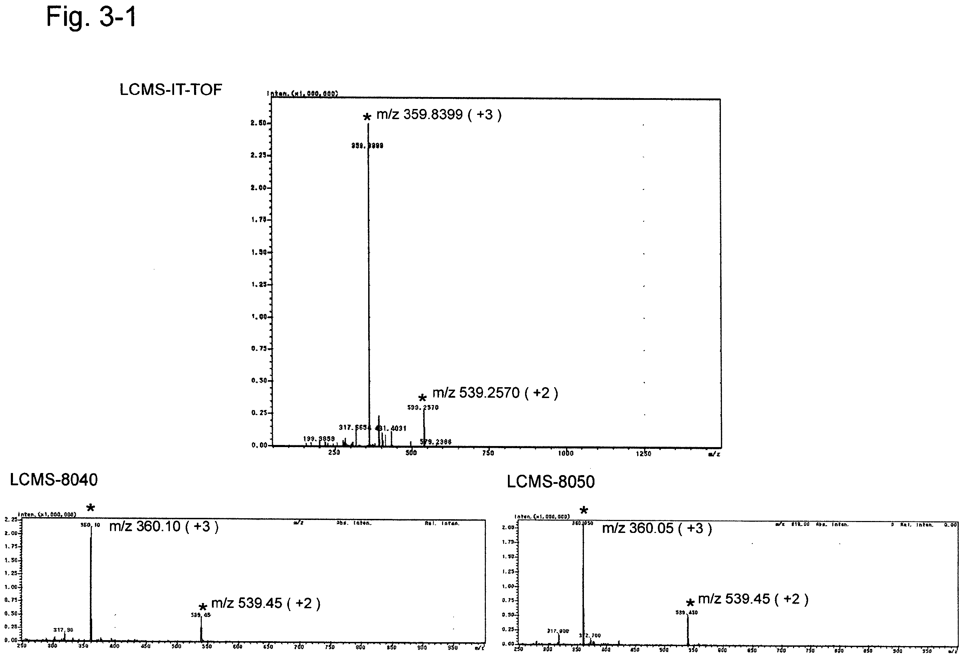

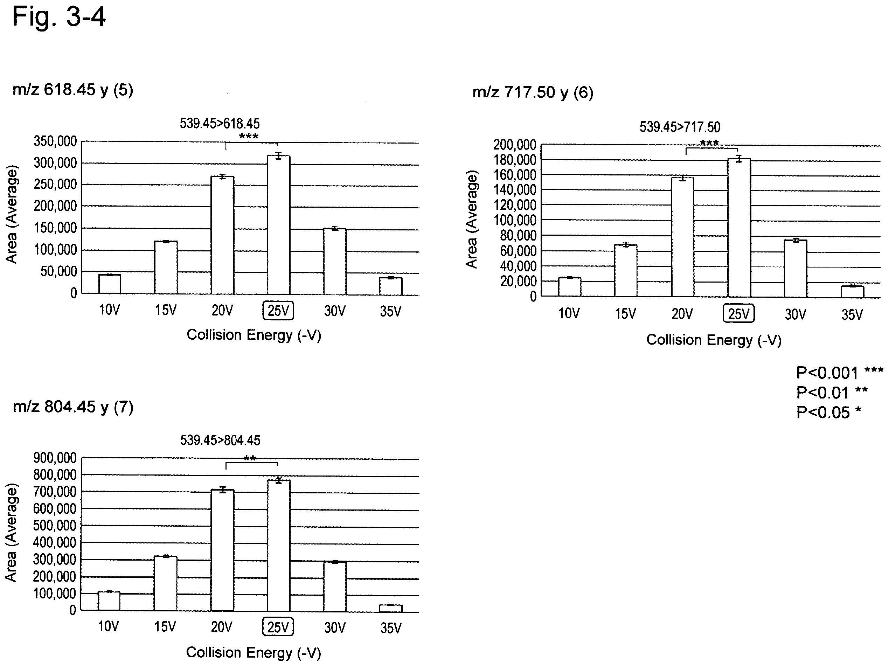

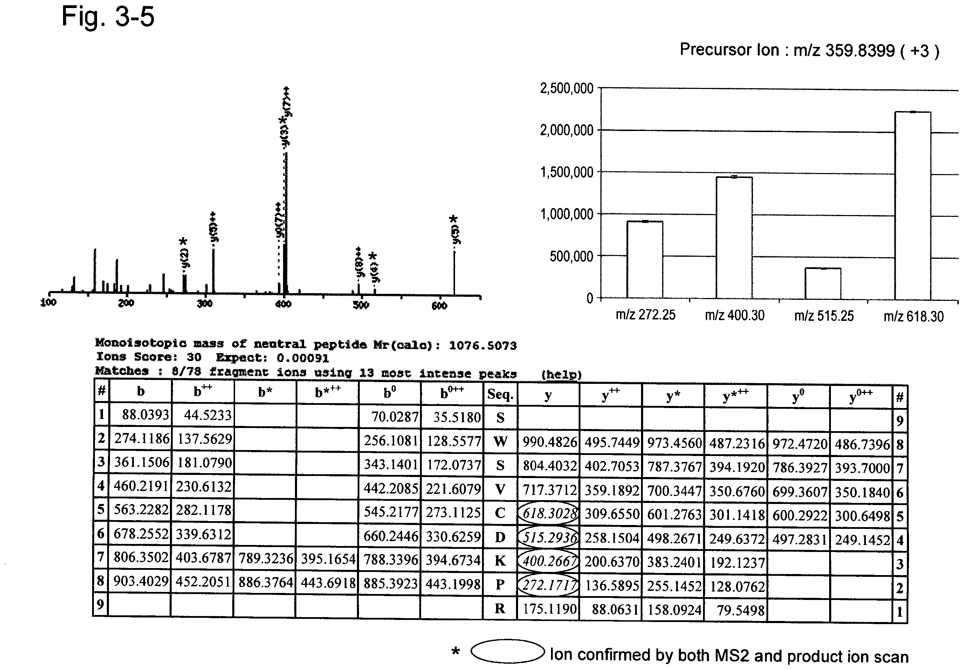

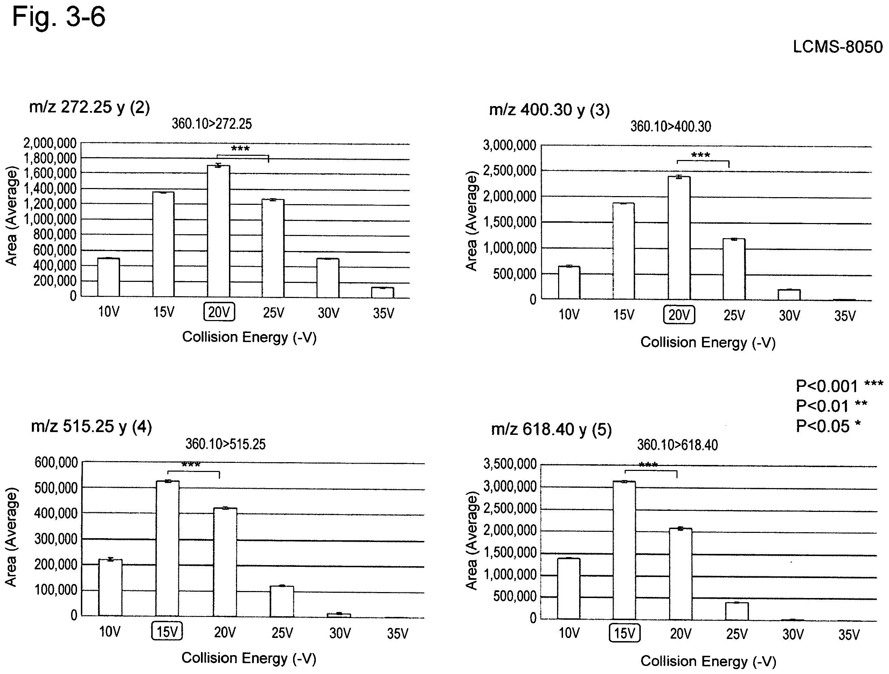

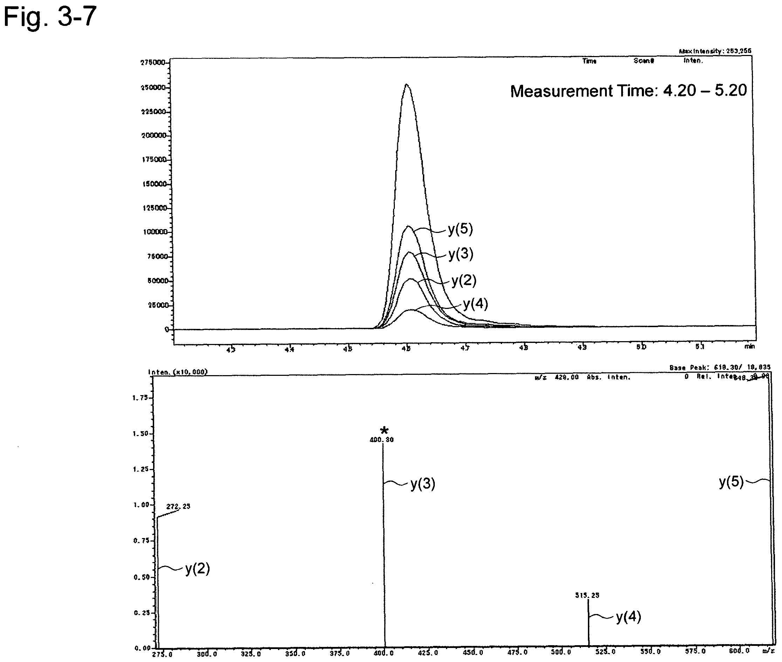

[Fig. 3-1] Fig. 3-1 shows results of a preliminary MS analysis and a Q3 scan analysis of a peptide P14 (SEQ ID NO: 24).

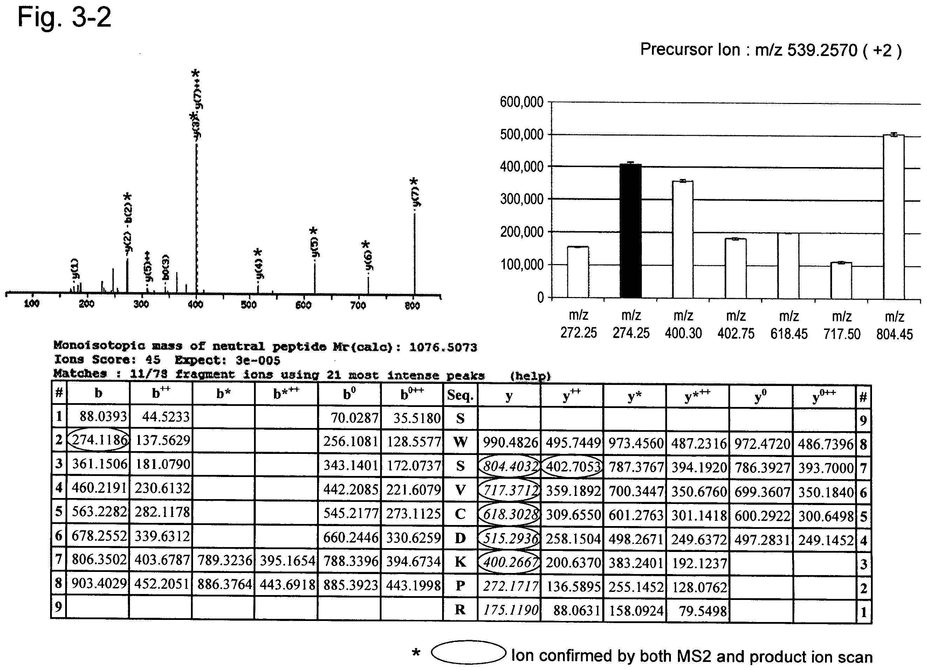

[Fig. 3-2] Fig. 3-2 shows MS2 analysis results of the peptide P14 (SEQ ID NO: 24) and peaks of expected product ions obtained by an analysis of a selected precursor ion and detected product ions.

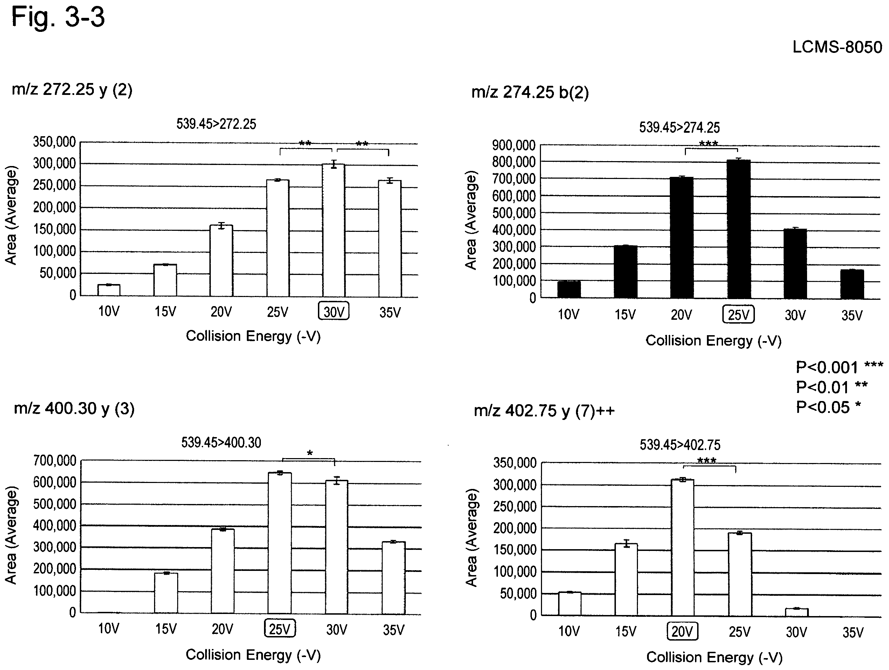

[Fig. 3-3] Fig. 3-3 shows examination results of optimization conditions of collision energies for detecting product ions to be used for analyzing the peptide P14 (SEQ ID NO: 24) (in a case where LCMS-8050 was used).
[Fig. 3-4] Fig. 3-4 shows examination results of optimization conditions of collision energies for detecting product ions to be used for analyzing the peptide P14 (SEQ ID NO: 24) (in a case where LCMS-8050 was used).
[Fig. 3-5] Fig. 3-5 shows MS2 analysis results of the peptide P14 (SEQ ID NO: 24) and peaks of expected product ions obtained by an analysis of a selected precursor ion and detected product ions.
[Fig. 3-6] Fig. 3-6 shows examination results of optimization conditions of collision energies for detecting product ions to be used for analyzing the peptide P14 (SEQ ID NO: 24) (in a case where LCMS-8050 was used).
[Fig. 3-7] Fig. 3-7 shows results of quantifying the peptide P14 (SEQ ID NO: 24) by detecting y(2), y(3), y(4) and y(5) ions under the optimization conditions.

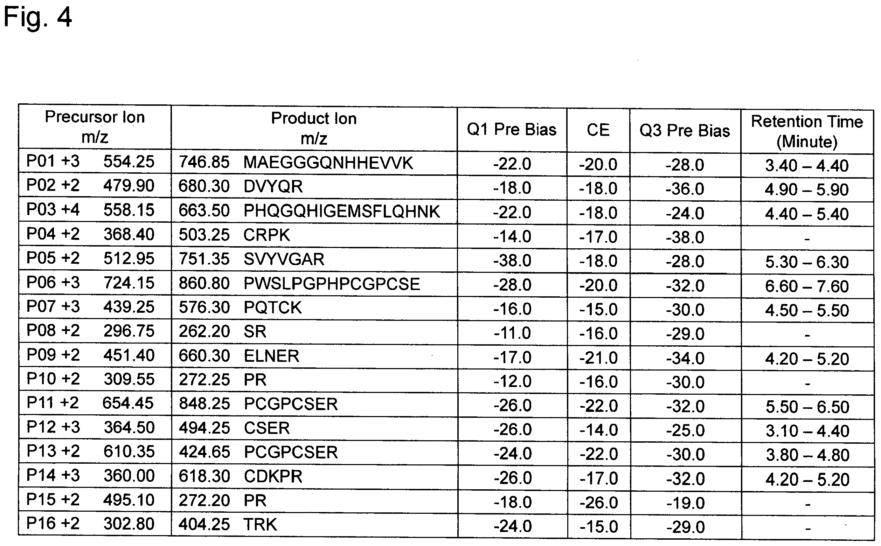

[Fig. 4] Fig. 4 shows an example of an suitable analysis condition for simultaneous determination of peptides P01 - P16.

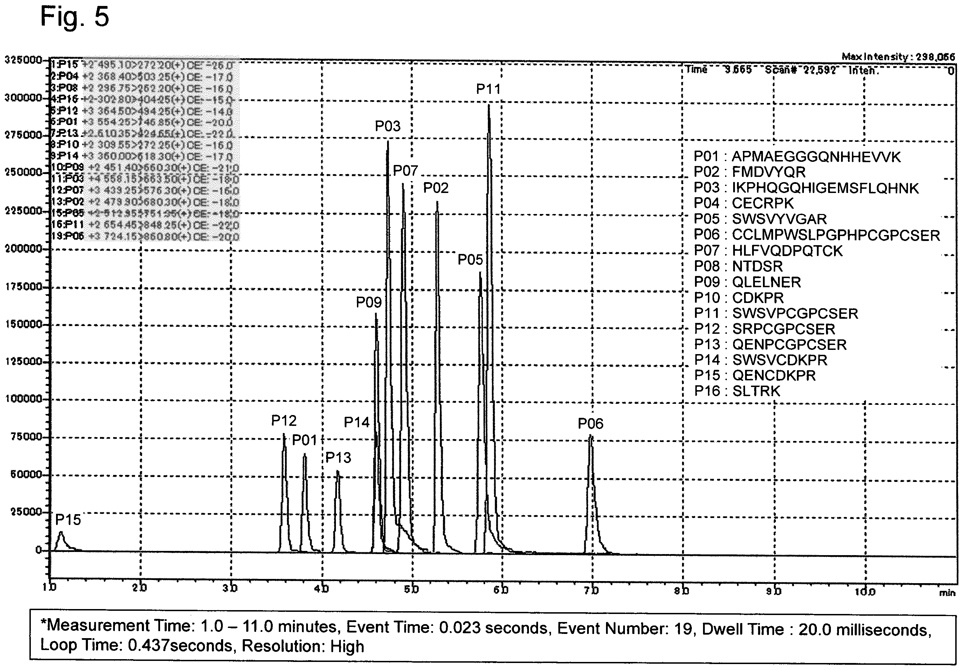

[Fig. 5] Fig. 5 shows an example of simultaneous determination of the peptides P01 - P16.

### [Method for Quantifying Variant]

Described herein is a method for parallel determination of variants of a protein having 2 or more variants using mass spectrometry. The method includes: protease-digesting a protein in a sample; detecting, using mass spectrometry, among peptides obtained by the protease digestion, 2 or more kinds of peptides having an amino acid sequence specific to each of the variants; and determining an amount of each of the variants in the sample based on a result of the mass spectrometry..

Here, a "variant" of a protein refers to a protein having a different amino acid sequence, for which the difference is due to deletion, substitution, addition, or the like with respect to a part of a protein having a certain amino acid sequence, and is usually called a "variant," a "mutant" or the like in the art. A variant may be a naturally occurring variant or an artificially produced variant.

Herein, it is assumed that 2 or more variants are mixed in the same sample. Therefore, in a case where only a "mutant-type" protein that is different from a "wild-type" protein is contained in a sample, the method described herein does not include detecting the "mutant-type" protein.

As a variant, an example is a splicing variant. As is well known in the art, when exons are ligated from primary transcription products (pre-mRNAs) generated in transcription from eukaryotic DNAs and mRNAs are generated, splicing variants refer to different proteins translated from multiple mRNAs having different sequences generated by splicing at different sites, selection of different exons, and the like. These are sometimes described as "isoforms" of the same protein. In the present specification, all possible proteins are described as "mutants" or "variants." Further, in a case where a protein generated by translation is subsequently modified or cleaved by processing or the like, as long as the modification products and the cleavage products can be mixed in a sample, the modification products and the cleavage products are included in the "mutants" or "variants" to be detected using the method described herein.

The "sample" is a sample taken from a subject who is assumed to have a specific protein, and, for example, can be a sample or the like derived from blood (whole blood, serum or plasma), body fluid such as saliva, pleural effusion and ascites, and tissue. A sample may contain a reagent added as needed. In order to efficiently obtain a protein to be analyzed from a sample such as blood, for example, an antibody specific to the protein may be used. For example, when the protein is VEGF-A, all VEGF-A can be collected by immunoprecipitation from serum of a patient using an anti-VEGF-A antibody such as bevacizumab, and a proportion of a VEGF-A variant contained therein can be quantified. VEGF-A and an antibody complex can be subjected to an LCMS analysis by protease-digestion after reductive alkylation.

A peptide to be detected using the method described herein is a peptide having an amino acid sequence specific to a variant among the peptides obtained by the protease digestion.

A protease that can be suitably used in the method described herein may be appropriately selected according to a kind of a protein to be quantified or identified using mass spectrometry, and is not limited. Examples of the protease include trypsin, chymotrypsin, lysyl endopeptidase (Lys-C), V8 protease, Asp N protease (Asp-N), Arg C protease (Arg-C), papain, pepsin, dipeptidyl peptidase, and the like. Two or more kinds of these proteases can be used in combination. Examples of a protease that can be more suitably used herein include trypsin, Lys-C, Glu-C, Asp-N, chymotrypsin and the like.

Among these proteases, trypsin is particularly preferably used herein. Trypsin has high substrate specificity and has Lys or Arg at a C terminal of a peptide after cleavage and thus can homogenize a charge amount and charge localization of a peptide, and is particularly suitable for preparation of a sample for mass spectrometry. Examples of the protease that can be suitably used in the method described herein include Trypsin Gold (manufactured by Promega), Trypsin TPCK-Treated (manufactured by Sigma), and the like.

The protease can also be used by being immobilized on a support such as activated carbon, a porous membrane, porous resin beads, metal particles and the like, when necessary. Procedures of the protease digestion for mass spectrometry are commonly performed in the art, and a person skilled in the art can appropriately determine a detailed reaction condition.

The method described herein allows presence and an amount of each variant in a sample to be determined by detecting a peptide having an amino acid sequence specific to the each variant. In the present specification, "an amino acid sequence specific to each variant" means an amino acid sequence that exists in some variants and does not exist in other variants. Therefore, "a peptide having an amino acid sequence specific to each variant" means a peptide that is obtained by protease digestion of one variant or some of multiple variants of a protein and enables quantification of the one variant or the some of the multiple variants based on existence thereof. In particular, a peptide having an amino acid sequence specific to one kind of a variant may sometimes be described as "a peptide having an amino acid sequence specific to only -" in the present specification.

Each variant may contain one "peptide having a specific amino acid sequence" or may contain two or more "peptides having a specific amino acid sequence." Depending on a protein, it may also be necessary to detect two or more such peptides in order to identify presence and an amount of a variant.

In addition to this, the method described herein may further include detecting a peptide having an amino acid sequence common to 2 or more variants. Such a peptide can be a peptide having an amino acid sequence common to all variants that are present in a sample. By detecting also such a peptide, an amount of information obtained from an analysis can be increased, and reliability of a detection result can be further increased. By detecting a peptide having an amino acid sequence common to 2 or more variants in addition to a peptide having an amino acid sequence specific to each variant, it can be confirmed that an intended protein can be reliably measured, and each variant can be quantified. Further, since a total amount of a protein can also be quantified, it is very important to quantify a peptide having an amino acid sequence that is common among variants.

Some variants themselves may sometimes not contain "a peptide having a specific amino acid sequence." For example, in a case where a variant obtained by deleting a part of a protein (variant) is produced, the deletion variant may sometimes not contain "a peptide having a specific amino acid sequence." In this case, based on a detection result of a peptide having an amino acid sequence specific to other variants and a detection result of a peptide having an amino acid sequence common to 2 or more variants, it is possible to quantify the above-mentioned variant that does not include "a peptide having a specific amino acid sequence." In a case where various variants coexist, it is possible that all multiple kinds of peptides obtained by protease digestion of a certain variant are peptides that are commonly contained with any other variant. In this case, in the present specification, in order to identify those peptides, they are described as "a peptide having an amino acid sequence common to (all) variants" and "a peptide having an amino acid sequence specific to (multiple kinds of) variants."

For example, in a case of simultaneous quantification of splicing variants of VEGF-A, by detecting a peptide having an amino acid sequence common to the variants in addition to a peptide having an amino acid sequence specific to each variant, an evidence that VEGF-A can be reliably measured and information such as an amount of each variant among them can be obtained, and further a total VEGF-A amount can also be referenced.

In the method described herein, two or more kinds of protease-digested peptides are subjected to mass spectrometry. A sample in which the protease-digested peptides are mixed can be directly subjected to mass spectrometry, for example, without separating the peptides by SDS-PAGE or the like. However, when necessary, removal of a used protease or the like can be performed.

### [Mass Spectrometry]

The method described herein allows identification and quantification of multiple variants to be simultaneously performed in parallel by analyzing, using mass spectrometry, a sample containing two or more kinds of peptides obtained above. For mass spectrometry, preferably, liquid chromatography mass spectrometry (LC-MS) is used.

For a purpose such as more reliably separating peptide fragments and improving analysis accuracy, a sample before being subjected to mass spectrometry is subjected to separation and concentration using liquid chromatography (LC). When separation of a sample is performed using LC, an eluate from LC may be directly ionized and subjected to mass spectrometry. An analysis can also be performed using LC/MS/MS or LC/MSn combining LC with tandem mass spectrometry. Further, the eluate from the LC may be collected once and then subjected to mass spectrometry. An LC column is not particularly limited, and a reverse phase column such as C30, C18, C8, and C4 generally used in a peptide analysis, a carrier for hydrophilic affinity chromatography, and the like can be appropriately selected and used.

Mass spectrometry can determine an amino acid sequence and thus can determine whether or not a peptide fragment is a peptide fragment derived from a specific protein. Further, based on a peak intensity, a concentration of a peptide fragment in a sample can be determined. In performing an analysis, when necessary, a sample may be used for the analysis after being subjected to treatments such as desalting, solubilization, extraction, concentration, and drying.

An ionization method in mass spectrometry is not particularly limited, and an electron ionization (EI) method, a chemical ionization (CI) method, a field desorption (FD) method, a fast atom collision (FAB) method, a matrix assisted laser desorption ionization (MALDI) method, an electrospray ionization (ESI) method, and the like can be adopted. A method for analyzing an ionized sample is also not particularly limited, and a method of a magnetic field deflection type, a quadrupole (Q) type, an ion trap (IT) type, a time of flight (TOF) type, a Fourier transform ion cyclotron resonance (FT-ICR) type, or the like can be appropriately determined according to the ionization method. Further, an MS/MS analysis or multistage mass spectrometry of MS3 or higher can also be performed using triple quadrupole mass spectrometer or the like.

A device that is suitable for being used in the method described herein is not particularly limited. Examples of the device include LCMS-8030, LCMS-8040, LCMS-8050, and LCMS-8060 (all manufactured by Shimadzu Corporation), and LCMS-IT-TOF and LCMS-Q-TOF (manufactured by Shimadzu Corporation).

Based on a mass spectrometry result, in order to identify a protein, an existing database can also be used. For example, various information such as identification of a protein candidate can be obtained by using a Mascot search (Matrix Science Corporation) to automatically perform attribution of assumed parent ion or fragment ion series from spectrum information obtained using mass spectrometry.

Further, it is also possible to identify a protein by identifying an amino acid sequence of a peptide fragment using multistage mass spectrometry or the like. When a peptide fragment having an amino acid sequence specific to a certain variant can be detected, a target variant can be identified and quantified.

The number of amino acid residues of a peptide to be detected is preferably about 5 - 30, and more preferably about 7 - 25. When the number of the amino acid residues is excessively small, column retention capacity becomes weak and purification becomes difficult, and it is difficult to be distinguished from a contaminant or a peptide fragment derived from another site of the same protein, and this can cause erroneous detection and the like. Further, when the number of the amino acid residues is excessively large, for reasons such as instability of a collection rate or that ionization becomes difficult, detection may become difficult and quantitativeness may decrease.

When concentration of each variant is quantified, an amount of the each variant can be calculated based on a peak area or a peak intensity of a detected peptide fragment ion (in the case of multistage MS, a fragment ion obtained by cleavage of a parent ion). For example, based on a correlation between a predetermined calibration curve and a peak area, or a correlation between a peak area derived from an internal standard added to a sample and a peak area derived from the sample, a concentration of a peptide fragment in the sample is calculated, and, based on the concentration of the peptide fragment, an amount and a concentration of each variant are calculated.

For detection of each variant, measurement of each variant can be performed with a measurement time in a range of several milliseconds - several tens of milliseconds, and a continuous analysis can be performed while switching channels. As a result, for purposes such as quantifying a protein in a sample and determining an abundance ratio of each variant, variants that can be present in a sample can be quantified all at once. Detection using mass spectrometry is fast and accurate, and a very large amount of information can be obtained in a short time. The number of variants that can be quantified in parallel using the method described herein is not particularly limited, but is two or more kinds, three or more kinds, four or more kinds, or five or more kinds, and can be 10 or more kinds, 15 or more kinds, or 20 or more kinds.

### [VEGF-A Variant]

A preferred embodiment described herein is a method for detecting and quantifying a splicing variant of a vascular endothelial growth factor (VEGF)-A.

It is known that there are about 10 kinds of splicing variants of VEGF-A, and it is believed that an effect is determined by a balance of an entire mixture of variants present in an individual human body.

Each amino acid sequence of VEGF-A variants can be obtained by referring to a database that can be accessed via the Internet, for example, the SwissProt database (http: //www.genome.jp/dbget-bin/www_bget?sp: VEGFA_HUMAN).

For selection of partial peptides suitable for a quantitative analysis of 10 kinds of variants 206, 189, 183, 165, 148, 145, 121, 165B, 121B and 111 (SEQ ID NOs: 1 - 10), the present inventors first compared amino acid sequences of these variants by sequence alignment. Results of the alignment are shown in Figs. 1-1 and 1-2.

In order to be able to quantify all VEGF-A variants at once, the present inventors examined a peptide candidate generated from each variant when trypsin is used as a protease. That is, in order to identifiably quantify each variant, fragments (peptides P01 - P16, SEQ ID NOs: 11 - 26) that have a sequence common to all variants and a sequence specific to one or several kinds of variants in the above alignment and are obtained by protease digestion were selected. The results are shown in Table 1.

### [Table 1]

**Table 1. Peptide targets for quantifying all VEGF-A variants at once**

| No | Peptide | SEQ ID NO. | 206 | 189 | 183 | 165 | 148 | 145 | 121 | 165B | 121B | 111 |
|---|---|---|---|---|---|---|---|---|---|---|---|---|
| P01 | APMAEGGGQNHHEVVK | 11 | ○ | ○ | ○ | ○ | ○ | ○ | ○ | ○ | ○ | ○ |
| P02 | FMDVYQR | 12 | ○ | ○ | ○ | ○ | ○ | ○ | ○ | ○ | ○ | ○ |
| P03 | IKPHQGQHIGEMSFLQHNK | 13 | ○ | ○ | ○ | ○ | ○ | ○ | ○ | ○ | ○ | ○ |
| P04 | CECRPK | 14 | ○ | ○ | ○ | ○ | ○ | ○ | ○ | ○ | ○ | |
| P05 | SWSVYVGAR | 15 | ○ | | | | | | | | | |
| P06 | CCLMPWSLPGPHPCGPCSER | 16 | ○ | | | | | | | | | |
| P07 | HLFVQDPQTCK | 17 | ○ | ○ | ○ | ○ | ○ | | | ○ | | |
| P08 | NTDSR | 18 | ○ | ○ | ○ | ○ | ○ | | | ○ | | |
| P09 | QLELNER | 19 | ○ | ○ | ○ | ○ | | | | ○ | | |
| P10 | CDKPR | 20 | ○ | ○ | ○ | ○ | | | | | ○ | ○ |
| P11 | SWSVPCGPCSER | 21 | | ○ | | | | | | | | |
| P12 | SRPCGPCSER | 22 | | | ○ | | | | | | | |
| P13 | QENPCGPCSER | 23 | | | | ○ | ○ | | | ○ | | |
| P14 | SWSVCDKPR | 24 | | | | | | ○ | | | | |
| P15 | QENCDKPR | 25 | | | | | | | ○ | | | |
| P16 | SLTRK | 26 | | | | | | | | ○ | | |

As can be understood from Table 1, the peptides P01 (SEQ ID NO: 11), P02 (SEQ ID NO: 12) and P03 (SEQ ID NO: 13) are peptides that are detected in all of the 10 kinds of the variants. Further, the peptides P07 (SEQ ID NO: 17) and P08 (SEQ ID NO: 18) are peptides that are detected in 6 kinds among the 10 kinds of the variants. In contrast, the peptides P05 (SEQ ID NO: 15), P06 (SEQ ID NO: 16), P11 (SEQ ID NO: 21), P12 (SEQ ID NO: 22), P14 (SEQ ID NO: 24), P15 (SEQ ID NO: 25), and P16 (SEQ ID NO: 26) are peptides that are respectively detected only in the variants 206, 206, 189, 183, 145, 121, and 165B.

That is, the peptides P05, P06, P11, P12, P14, P15, and P16 are peptides having amino acid sequences that respectively specific to only the variants 206, 206, 189, 183, 145, 121, and 165B, and the peptides P01 - P03 are peptides that each have an amino acid sequence common to the 10 kinds of the variants. The peptides P04, P07 - P10, and P13 are peptides that each have an amino acid sequence specific to some of the 10 kinds of the variants.

Therefore, by detecting the peptides P01 - P16 in combination, all of the variants 206, 189, 183, 165, 148, 145, 121, 165B, 121B and 111 can be identified and quantified. That is, described herein is a method in which the variants include 2 or more selected from the following splicing variants of VEGF-A: 206 (SEQ ID NO: 1), 189 (SEQ ID NO: 2), 183 (SEQ ID NO: 3), 165 (SEQ ID NO: 4), 148 (SEQ ID NO: 5), 145 (SEQ ID NO: 6), 121 (SEQ ID NO: 7), 165b (SEQ ID NO: 8), 121b (SEQ ID NO: 9), and 111 (SEQ ID NO: 10). Although not particularly limited, this embodiment includes, for example, detecting 1 or more of the peptides (P01 - P16) having amino acid sequences shown in SEQ ID NOs: 11 - 26.

To further describe the method using the above embodiment, in a case where variants contained in a sample are already known, or in a case where variants to be analyzed are specific variants, as shown in Table 1, it is sufficient to detect only peptides for each variant.

On the other hand, in a case where variants contained in a sample are unknown and it is desired to detect the variants all at once, these peptides P01 - P16 can be simultaneously quantified. Based on the detection results of the peptides P01 - P16, an amount of each variant in the sample can be calculated.

For example, the peptide P14 (SEQ ID NO: 24) is a peptide having an amino acid sequence specific to only the variant 145 (SEQ ID NO: 6). Therefore, the existence and the amount of the variant 145 in the sample can be determined by detecting the peptide P14 with or without detecting the peptides P01, P02, P03 and P04.

Further, the peptide P16 (SEQ ID NO: 26) is a peptide having an amino acid sequence specific to only the variant 165B (SEQ ID NO: 8). Therefore, the existence and the amount of the variant 165B in the sample can be determined by detecting the peptide P16 with or without detecting the peptides P01 - P04, P07 - P09 and P13.

On the other hand, each of the variants 165, 148, 121B and 111 does not contain a peptide specific to only the each of the variants. However, the existence and the amounts of these variants can be determined based on detection results of peptides that each have an amino acid sequence specific to some of the variants, or, in addition to that, based on detection results of peptides that each have an amino acid sequence common to all the variants.

For example, the variant 165 (SEQ ID NO: 4) can generate, by trypsin digestion, the peptides P01 - P03 that each have an amino acid sequence common to the 10 kinds of the variants, and the peptides P04, P07 - P10 and P13 that each have an amino acid sequence specific to some of the variants. Further, the variant 165 is similar to the variant 165B in that the peptides P01 - P04, P07 - P09 and P13 are included, and is different from the variant 165B in that the peptide P10 is included and the peptide P16 is not included. Therefore, the presence and the amount of the variant 165 in the sample can be determined by detecting the peptides P04, P07, P08, P09, P10 and P13 in addition to the peptides P01, P02, P03 and based on the detection results.

Peptides obtained by trypsin digestion of the variant 111 (SEQ ID NO: 10) are the peptides P01 - P03 that each have an amino acid sequence common to the 10 kinds of the variants, and the peptide P10 that has an amino acid sequence specific to the 6 kinds of the variants (206, 189, 183, 165, 121B and 111). Peptides obtained by trypsin digestion of the variant 121B (SEQ ID NO: 9) are the above 4 kinds of the peptides obtained by trypsin digestion of the variant 111 and the peptide P04. Therefore, the presence and the amounts of these variants in the sample also can be determined by detecting these peptides and based on the detection results.

Further, by quantifying the peptides P01 - P03 that each have an amino acid sequence common to all the variants, the total VEGF-A amount can be quantified, and that the peptides P01 - P03 can be simultaneously measured can also be used as verification data of that VEGF- A is undoubtedly measured.

### [Optimization of Quantification Condition]

The method described herein is to identify and quantify each variant in a sample, and detection is directed to peptides generated by protease digestion of the sample. However, in order to establish a detailed condition for the detection of each peptide, it is possible to chemically synthesize a peptide based on amino acid information acquired in advance. Synthesis of a peptide can be performed based on a method commonly used in the art.

A main objective of the present disclosure is to establish a method that allows detection to be promptly performed in a clinical setting. Therefore, the present inventors examined optimization of a quantification condition for each synthesized peptide. The following description is based on an experiment performed on VEGF-A. However, a person skilled in the art can understand that detection of variants of other proteins can be performed by the same procedure.

### [1. Identification of Structure, Confirmation of MS/MS Ion]

For a synthesized peptide, although it is not an essential step, first, it is preferable to perform confirmation of a structure. For example, it is preferable to perform MS and MS/MS ion confirmation and a detailed structure analysis using LCMS-IT-TOF (manufactured by Shimadzu Corporation).

For the above purpose, for example, the present inventors performed a Flow inject MS/MS auto analysis.

A private database incorporating VEGF- A variants is created. On the other hand, attribution of fragment ion series can be performed using a Mascot MS/MS ion search that performs a Mascot search. For a peptide that was not involved in MS/MS in the auto analysis, manual MS/MS analysis can be performed again using a parent ion as an indicator.

Further, as a result of a Mascot search, for a peptide fragment that has a small chain length and is not involved in criteria, collation of a fragment ion can be performed using a Fragment Ion Calculator (http://db.systemsbiology.net:8080/proteomicsToolkit/FragIonServlet.html).

The following Table 2 shows an example of mass spectrometry conditions that can be performed for structure identification of a peptide and MS/MS ion confirmation.

### [Table 2]

**Table 2. Identification of structure, confirmation of MS/MS ions**

| LC Conditions (flow inject) | | MS measurement conditions | |
|---|---|---|---|
| Inject: | 2 pmol / µL 1 µL | Segment 1 | Positive ion mode |
| T.Flow: | 0.2 mL / minute | Auto analysis | Event 1 (MS1) Event 2 (MS2) |
| B.Conc: | 20% | MS program: | 0.00 minutes Flow path switching Introduction side |
| A pump: | 0.1% FA / DDW | | 1.00 minutes Flow path switching Drain side |
| | (wako DDW 3 L + FLUKA FA 3 mL) | MS measurement time:: | 0.00 - 1.00 minutes |
| B pump: | 0.1% FA/ACN | Measurement m/z: | 100 - 1500 (MS) |
| | (wako ACN 1 L + FLUKA FA 1 mL) | | 200 - 1300 (MS2 precursor ion)) |
| | | | 50 - 2000 (MS2 precursor ion)) |
| | | Repeat: | 3 (Initial value) |
| | | Ion storage time: | MS 30 milliseconds |
| | | | MS 270 milliseconds |
| | | CID energy: | 50% (usually 30%) |

The present inventors performed valence identification a parent ion detected under the above conditions for each of the synthesized peptides P01 - P16. The results are shown in Table 3. In Table 3, m/z values of calculated parent ions are shown; parent ions detected using both LCMS-IT-TOF suitable for structure determination and LCMS-8050 suitable for quantification (both LCMS-IT-TOF and LCMS-8050 are manufactured by Shimadzu Corporation) are indicated in bold and underlined; and parent ions detected using only one of LCMS-IT-TOF and LCMS-8050 are underlined.

### [Table 3]

**Table 3. Valence identification of detected VEGF-A peptides**

| No | Peptide | +1 | +2 | +3 | +4 | +5 |
|---|---|---|---|---|---|---|
| P01 | APMAEGGGQNHHEVVK | 1660.786 | ***830.8894*** | ***554.2596*** | ***415.9447*** | 332.9558 |
| P02 | FMDVYQR | 958.445 | ***480.2226*** | *320.4817* | 240.6113 | 192.6890 |
| P03 | IKPHQGQHIGEMSFLQHNK | 2229.135 | 1115.5673 | ***744.0448*** | ***558.2836*** | ***446.8269*** |
| P04 | CECRPK | 735.328 | ***368.1681*** | ***245.7813*** | 184.5880 | 147.8640 |
| P05 | SWSVYVGAR | 1024.521 | ***512.7648*** | 342.1791 | 256.8863 | 205.7028 |
| P06 | CCLMPWSLPGPHPCGPCSER | 2169.916 | 1085.4622 | ***723.9774*** | 543.2350 | 434.7817 |
| P07 | HLFVQDPQTCK | 1315.646 | ***658.3274*** | ***439.2209*** | 329.6676 | 263.9262 |
| P08 | NTDSR | ***592.269*** | **296.6385** | 198.0949 | 148.8232 | 119.2523 |
| P09 | QLELNER | 901.474 | ***451.2411*** | 301.1634 | 226.1245 | 181.0933 |
| P10 | CDKPR | ***618.303*** | ***309.6556*** | *206.7731* | 155.3318 | 124.4591 |
| P11 | SWSVPCGPCSER | 1307.551 | ***654.2796*** | 436.5224 | 327.6437 | 262.3087 |
| P12 | SRPCGPCSER | 1091.472 | ***546.2403*** | ***364.4961*** | 273.6241 | 219.0930 |
| P13 | QENPCGPCSER | 1219.483 | ***610.2458*** | *407.1665* | 305.6268 | 244.6952 |
| P14 | SWSVCDKPR | 1077.515 | ***539.2615*** | ***359.8436*** | 270.1347 | 216.3015 |
| P15 | QENCDKPR | 989.447 | ***495.2277*** | ***330.4878*** | 248.1178 | 198.6879 |
| P16 | SLTRK | ***604.378*** | ***302.6931*** | *202.1313* | 151.8505 | 121.6741 |

### [2-1. Optimization of MRM Condition in Device (LCMS-8040) to be used]

A mass spectrometry condition can vary depending on a device to be used. Therefore, it is necessary to determine an optimum MRM condition in a device to be actually used in measurement.

The present inventors subsequently performed, for example, a Q3 scan and a product ion scan using LCMS-8040 (manufactured by Shimadzu Corporation) which is also suitable for quantification.

Comparing to ions attributed using LCMS-IT-TOF, a precursor ion and a product ion to be used for an MRM gate are selected based on a calculated value and a measured value. The product ion is preferably obtained from a y ion series having sequence specificity. However, when selection of a y ion is difficult, in some cases, a b ion series can also be a transition of a quantification target.

It is preferable to preferentially select a sequence-specific fragment. For example, a fragment having a large structural strain such as a C-terminal side fragment of Pro can be used as an indicator.

An optimum collision energy can be obtained using Flow inject MRM.

Table 4 shows an example of MRM conditions optimized for detection of VEGF-A variants.

### [Table 4]

**Table 4. Optimization of LCMS-8040 MRM conditions**

| LC conditions (flow inject) | | MS measurement conditions |
|---|---|---|
| Inject: | 2 pmol / µL 1 µL (MRM 5 µL) | Positive ion mode |
| T.Flow: | 0.2 mL / minute | Q3 scan |
| B.Conc: | 20% | Measurement start m/z 250.00 end m/z 1000.00 |
| A pump: | 0.1% FA / DDW | Event time: 1.000 seconds |
| | (wako DDW 3 L + FLUKA FA 3 mL) | Product ion scan |
| B pump: | 0.1% FA / ACN | Measurement start m/z 100.00 end m/z 1000.00 |
| | (wako ACN 1 L + FLUKA FA 1 mL) | Event time: 0.500 seconds |
| Time program: | 0.00 minutes Valve switching MS side | Scan speed: 1875 u/s |
| | 1.00 minutes Valve switching drain side | Loop time: 2 seconds |
| Measurement time: | 0.00 - 1.50 minutes | Collision energy: -10 ∼ -25 V |
| | | MRM |
| | | Event time: 0.412 seconds |
| | | Loop time: 0.824 seconds |
| | | Dwell Time: 100 milliseconds |
| | | Collision energy: -10 ∼ -35 V |

### [2-2. Optimization of MRM Condition in Device (LCMS-8050) to be used]

The present inventors subsequently confirmed whether or not the MRM gate candidate determined using LCMS-8040 can also be used in LCMS-8050. Further, optimization of a Q1 Pre Bias voltage, a collision energy, a Q3 Pre Bias voltage was performed.

Here, resolution was changed from "unit" to "high" and m/z of a precursor ion was optimized in a high resolution MRM mode.

Table 5 shows an example of MRM conditions optimized for detection of VEGF-A variants.

By using three kinds of LCMS, confirmation of stability, reproducibility and model dependence of a generated ion was performed, and that MRM transition reliably functions was confirmed.

### [Table 5]

**Table 5. Optimization of LCMS-8050 MRM conditions**

| LC conditions (flow inject) | | MS measurement conditions |
|---|---|---|
| Inject: | 200 fmol / µL 5 µL (1 pmol upon injection) | Positive ion mode |
| T.Flow: | 0.2 mL / minute | Q3 scan |
| B.Conc: | 20% | Measurement start m/z 250.00 end m/z 1000.00 |
| A pump: | 0.1% FA / DDW | Event time: 1.000 seconds |
| | (wako DDW 3 L + FLUKA FA 3 mL) | Product ion scan |
| B pump: | 0.1% FA / ACN | Measurement start m/z 100.00 end m/z 1000.00 |
| | (wako ACN 1 L + FLUKA FA 1 mL) | Event time: 0.500 seconds |
| Time program: | 1.50 minutes Controller stop | Scan speed 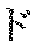 : 1875 u/s |
| | No valve switching | Loop time: 2 seconds |
| Measurement time: | 0.00 - 1.50 minutes | Collision energy: -10 ∼ -25 V |
| | | MRM |
| | | Event time: 0.412 seconds |
| | | Loop time: 0.824 seconds |
| | | Dwell Time: 50 milliseconds |
| | | Collision energy: -10 ∼ -35 V |

### [3. Determination of Column Retention time, MRM Analysis]

Subsequently, determination of a retention time and MRM measurement under the determined condition were performed using an L-column2 (2 µm, 2.1 × 50 mm (Chemical Substance Evaluation and Research Organization, CERI)) of LCMS-8050. LC conditions used are shown below.

### [LC Conditions]

Inject: 200 fmol/µL, 5 µL (1 pmol upon injection)
Flow rate: 0.m mL/minute
B concentration: 1 - 40%
A: 0.1% formic acid / DDW (wako DDW 3 L + FLUKA FA 3 mL)
B: 0.1% formic acid / acetonitrile (wako ACN 1 L + FLUKA FA 1 mL)
Time program: 1.00 minutes controller event 2 (Flow MS side)
2.00 minutes pump B.Conc 1%
10.00 minutes pump B.Conc 40%
11.00 minutes pump B.Conc 98%
11.00 minutes controller event 0 (Flow Drain side)
13.00 minutes pump B.Conc 98%
13.50 minutes pump B.Conc 1%
15.00 minutes pump B.Conc 1%
15.00 minutes Controller Stop
Measurement time: 1.00 - 11.00 minutes

Table 6 shows examples of measurement fragments that are respectively suitably selected for the peptides P01 - P16 (SEQ ID NOs: 11-26) under optimized conditions. Table 6 shows m/z values and a retention time of one kind of a fragment ion for each of of the peptides. By detecting these fragment ions, detection of the peptides P01 - P16 can be performed.

### [Table 6]

**Table 6. Optimized conditions for quantifying VEGF-A variants (MRM transition for quantification and retention times of peptides)**

| No | Peptide | Valence | Parent ion m/z | Fragment ion m/z | Measurement fragment | Retention time [minute] |
|---|---|---|---|---|---|---|
| P01 | APMAEGGGQNHHEVVK | +3 | 554.25 | 746.85 | MAEGGGQNHHEVVK | 3.30 - 4.30 |
| P02 | FMDVYQR | +2 | 479.90 | 680.30 | DVYQR | 4.80 - 5.80 |
| P03 | IKPHQGQHIGEMSFLQHNK | +4 | 558.15 | 663.50 | PHQGQHIGEMSFLQHNK | 4.30 - 5.30 |
| P04 | CECRPK | +2 | 368.40 | 503.25 | CRPK | ND |
| P05 | SWSVYVGAR | +2 | 512.95 | 751.35 | SVYVGAR | 5.40 - 6.40 |
| P06 | CCLMPWSLPGPHPCGPCSER | +3 | 723.97 | 860.39 | PWSLPGPHPCGPCSE | 6.40 - 7.60 |
| P07 | HLFVQDPQTCK | +3 | 439.25 | 576.30 | PQTCK | 4.40 - 5.40 |
| P08 | NTDSR | +2 | 296.75 | 262.20 | SR | ND |
| P09 | QLELNER | +2 | 451.40 | 660.30 | ELNER | 4.10 - 5.10 |
| P10 | CDKPR | +2 | 309.55 | 272.25 | PR | ND |
| P11 | SWSVPCGPCSER | +2 | 654.45 | 848.25 | PCGPCSER | 5.40 - 6.40 |
| P12 | SRPCGPCSER | +3 | 364.50 | 494.25 | CSER | 3.20 - 4.40 |
| P13 | QENPCGPCSER | +2 | 610.35 | 424.65 | PCGPCSER | 3.70 - 4.70 |
| P14 | SWSVCDKPR | +3 | 360.00 | 618.30 | CDKPR | 4.00 - 5.00 |
| P15 | QENCDKPR | +2 | 495.10 | 272.20 | PR | 1.00 - 1.50 |
| P16 | SLTRK | +2 | 302.80 | 303.20 | RK | ND |

Alternatively, the method described herein also allows two or more kinds of fragment ions to be selected and simultaneously detected for detection of the peptides P01 - P16. Table 7 summarizes optimized analysis conditions for quantification of each of the peptides P01 - P16.

### [Table 7]

**Table 7. Optimized conditions for quantifying VEGF-A variants (MRM transition)**

| No | Peptide sequence | Parent ion m/z | Fragment ion m/z | Q1 Pre Bias [V] | CE [V] | Q3 Pre Bias [V] |
|---|---|---|---|---|---|---|
| P01 | APMAEGGGQNHHEVVK | 554.25 | 581.30 | -22.0 | -21.0 | -30.0 |
| | | | 645.80 | -22.0 | -20.0 | -34.0 |
| | | | 681.35 | -22.0 | -20.0 | -26.0 |
| | | | 746.85 | -22.0 | -20.0 | -28.0 |
| P02 | FMDVYQR | 479.90 | 466.30 | -18.0 | -18.0 | -23.0 |
| | | | 565.25 | -18.0 | -22.0 | -40.0 |
| | | | 680.30 | -18.0 | -18.0 | -36.0 |
| | | | 811.35 | -18.0 | -20.0 | -30.0 |
| P03 | IKPHQGQHIGEMSFLQHNK | 558.15 | 663.50 | -22.0 | -18.0 | -24.0 |
| | | | 261.15 | -22.0 | -27.0 | -18.0 |
| | | | 398.20 | -22.0 | -26.0 | -29.0 |
| | | | 631.15 | -22.0 | -21.0 | -32.0 |
| P04 | CECRPK | 368.40 | 244.15 | -14.0 | -24.0 | -27.0 |
| | | | 400.35 | -14.0 | -18.0 | -29.0 |
| | | | 503.25 | -14.0 | -17.0 | -38.0 |
| | | | 632.30 | -14.0 | -18.0 | -32.0 |
| P05 | SWSVYVGAR | 512.95 | 303.20 | -38.0 | -17.0 | -22.0 |
| | | | 402.30 | -38.0 | -20.0 | -29.0 |
| | | | 565.25 | -38.0 | -18.0 | -40.0 |
| | | | 751.35 | -38.0 | -18.0 | -28.0 |
| P06 | CCLMPWSLPGPHPCGPCSER | 723.97 | 618.75 | -28.0 | -29.0 | -40.0 |
| | | | 718.80 | -28.0 | -28.0 | -38.0 |
| | | | 848.25 | -28.0 | -36.0 | -32.0 |
| | | | 860.80 | -28.0 | -20.0 | -32.0 |
| P07 | HLFVQDPQTCK | 439.25 | 288.65 | -16.0 | -14.0 | -20.0 |
| | | | 479.30 | -18.0 | -16.0 | -24.0 |
| | | | 576.30 | -16.0 | -15.0 | -30.0 |
| | | | 691.30 | -16.0 | -12.0 | -38.0 |
| P08 | NTDSR | 296.75 | 262.20 | -11.0 | -16.0 | -29.0 |
| | | | 377.15 | -11.0 | -10.0 | -27.0 |
| | | | 478.25 | -11.0 | -10.0 | -17.0 |
| P09 | QLELNER | 451.40 | 304.20 | -17.0 | -17.0 | -22.0 |
| | | | 418.25 | -17.0 | -17.0 | -30.0 |
| | | | 531.25 | -17.0 | -20.0 | -40.0 |
| | | | 660.30 | -17.0 | -21.0 | -34.0 |
| P10 | CDKPR | 309.55 | 272.25 | -12.0 | -16.0 | -30.0 |
| | | | 400.30 | -12.0 | -17.0 | -29.0 |
| | | | 515.30 | -12.0 | -16.0 | -26.0 |
| | | | 258.15 | -12.0 | -14.0 | -18.0 |
| P11 | SWSVPCGPCSER | 654.45 | 648.20 | -26.0 | -35.0 | -24.0 |
| | | | 751.20 | -26.0 | -32.0 | -40.0 |
| | | | 848.25 | -26.0 | -22.0 | -32.0 |
| | | | 1034.45 | -26.0 | -23.0 | -40.0 |
| P12 | SRPCGPCSER | 364.50 | 296.25 | -27.0 | -12.0 | -21.0 |
| | | | 391.25 | -27.0 | -14.0 | -28.0 |
| | | | 494.25 | -26.0 | -14.0 | -25.0 |
| | | | 591.15 | -27.0 | -14.0 | -32.0 |
| P13 | QENPCGPCSER | 610.35 | 424.65 | -24.0 | -22.0 | -30.0 |
| | | | 648.30 | -24.0 | -22.0 | -34.0 |
| | | | 848.25 | -24.0 | -25.0 | -32.0 |
| | | | 962.30 | -24.0 | -24.0 | -36.0 |
| P14 | SWSVCDKPR | 360.00 | 272.25 | -26.0 | -20.0 | -19.0 |
| | | | 400.30 | -26.0 | -19.0 | -29.0 |
| | | | 515.25 | -26.0 | -18.0 | -38.0 |
| | | | 618.30 | -26.0 | -17.0 | -32.0 |
| P15 | QENCDKPR | 495.10 | 272.20 | -18.0 | -26.0 | -19.0 |
| | | | 400.30 | -18.0 | -24.0 | -30.0 |
| P16 | SLTRK | 302.80 | 259.25 | -24.0 | -15.0 | -29.0 |
| | | | 303.20 | -24.0 | -9.0 | -21.0 |
| | | | 404.25 | -24.0 | -15.0 | -29.0 |
| | | | 517.25 | -24.0 | -16.0 | -38.0 |

Other examples of quantification of a protein using the method described herein are not limited, and include, for example, quantification of an angiotensin variant. Angiotensin is a peptide generated from angiotensinogen by enzymatic degradation, and there are four (I - IV) kinds of variants. Among these variants, although the angiotensins II - IV have a blood pressure increasing effect, it is known that the angiotensin I does not have a blood pressure increasing effect. Therefore, for the method described herein, a peptide suitable for quantification of each variant using mass spectrometry can be selected based on amino acid sequences of angiogenesinogen (SwissProt: P01015) and the angiotensins I - IV.

### [Kit]

The present disclosure further provides a kit for use in executing the method described herein using high performance liquid chromatograph mass spectrometry (LC-MS), the kit including: a protease; a reaction container for digesting the protein by bringing the protein and the protease into contact with each other; a buffer solution for causing a digestion reaction due to the protease to occur; and a set of internal standard peptides having amino acid sequences specific to the variants.

Measurement using mass spectrometry enables a very high-precision analysis. On the other hand, proper sample preparation and setting of appropriate analysis conditions are very important. For example, in order to more conveniently obtain an accurate examination result at a clinical site, described herein is a kit that can be used for implementing the above method.

The reaction container included in the kit described herein is not particularly limited as long as the reaction container is a container capable of allowing a protein (as a subject to be analyzed) and a protease to be in contact with each other in a liquid phase. However, considering an operation after detection using mass spectrometry, the reaction container is preferably a micro tube or a plate. Considering reaction processes such as mixing using a vortex or a rotator performed for a reaction and filtration for separation of a peptide and a protease after the reaction, a person skilled in the art can envision an appropriate reaction container.

The buffer solution included in the kit described herein is introduced into the reaction container together with a sample, which contains the protein, and a protease, and is for causing a digestion reaction by the protease to occur, and provides a reaction condition suitable for protease digestion. A reaction condition can be suitably determined depending on a protease and the like to be selected, and composition of the buffer solution can also be suitably determined.

The kit described herein can further include a filtration membrane for removing the protease and the like after the protease digestion reaction and extracting a product of the digestion reaction together with the buffer solution.

The kit described herein can further include an instruction manual describing how to use the kit and/or a mass spectrometry condition for detecting each variant.

The kit described herein includes a set of internal standard peptides. The internal standard peptides provide more reliable analysis results by being analyzed at the same time as a specimen or separately under the same condition as the specimen. The internal standard peptides are peptides that each contain an amino acid sequence specific to a variant as a measurement target and that have amino acid sequences that can be generated by digestion by the protease included in the kit described herein. The internal standard peptides can each be labeled, for example, using a stable isotope. In this case, since a mass spectrometry quantification condition can be slightly different as compared to an isotope-free peptide, it is preferable to enclose a quantification condition for an internal standard.

For example, when a measurement target is a splicing variant of VEGF-A, a set of peptides having the amino acid sequences of SEQ ID NOs: 11 - 26 are included as an internal standard peptides.

By using the kit described herein, a pretreatment such as preparation of a protease digestion fragment peptide and operation of mass spectrometry can be simplified and automation by using a device can also be easily achieved. In particular, when a protease such as trypsin is provided as a component of the kit in a form of an immobilized enzyme, the peptide preparation operation can be further simplified.

The kit can further contain reagent quality assurance data (atomic purity measurement result and the like), and can contain various other reagents and the like. Optional components of the kit are not particularly limited.

### [Recording Medium]

The present disclosure further provides a computer readable recording medium that is for use in the method described herein and in which data for executing mass spectrometry is stored. The data is not limited, but includes data of a parent ion, a fragment ion, an expected retention time, and voltages at triple quadrupoles (first quadrupole, second quadrupole, and third quadrupole) with respect to 1 or more peptides having amino acid sequences specific to variants.

The expected retention time, the voltage data, and the like are numerical values that vary depending on a device to be used and a measurement condition and the like, and are preferably provided in accordance with the device. Further, to facilitate understanding by a person skilled in the art, for a numerical value that varies depending on a condition, it is preferable to also provide a variation range of the numerical value.

The recording medium may be of any form, and is not particularly limited. Examples of the recording medium include disks and memories capable of magnetically or optically recording information.

More specifically, the above method package can include, for example, the following information and software functions.
- Optimized (*) parent ion m/z value
- Optimized fragment ion m/z value
- Optimized Q1 pre bias voltage value
- Optimized Q2 collision energy voltage value
- Optimized Q3 pre bias voltage value
- Expected retention time and mass spectrometry time of a target ion
- Quantitative value conversion formula
- Analysis result report output function
(*): Each condition item was actually measured, and one with the highest ionic strength and a most reproducible m/z value is adopted, and this is taken as an optimum value.

For example, the conditions/parameters described in the above Table 6 and/or Table 7 can be included as data for quantification of the splicing variants of VEGF-A.

### [Method Package]

The present disclosure further provides a method package for parallel detection of a protein variant using high performance liquid chromatograph mass spectrometry (LC-MS), the method package including the above-described recording medium, and an instruction manual of the recording medium.

In the present specification, the term "method package" means independently distributable package that includes, in a readable form, an analysis condition of liquid chromatography mass spectrometry with respect to a specific analysis target. By importing the data contained in the method package into LC-MS, it is possible to perform an analysis with an optimum measurement condition obtained after detailed examination.

As described above, while measurement using mass spectrometry enables very high-precision analysis, analysis conditions can be completely different depending on target ions. Therefore, although setting appropriate analysis conditions is very important, it is extremely difficult, and enormous time is required for setting the conditions. By preparing these conditions beforehand, convenience of a user who actually performs mass spectrometry can be improved. The present applicant has so far provided method packages for these analyses using LC-MS in order to allow a user to more easily perform mass spectrometry of, for example, agricultural chemicals or animal medicines.

An example of the above-described recording medium or method package is one that includes only information limited to a specific variant. Therefore, when variants to be analyzed are, for example, splicing variants of VEGF-A, a recording medium or a method package in which analysis conditions suitable for the splicing variants are described can be provided.

In this case, the data included in the recording medium can be related to, for example, analysis conditions with respect to 1 or more peptides having amino acid sequences shown in SEQ ID NOs: 11 - 26.

The method package may describe data common to multiple mass spectrometers or may describe various data suitable for an analysis using a specific mass spectrometer.

The recording medium or the method package can be provided together with the above-described kit or separately from the kit.

### [Examples]

The present invention is further described in detail based on the following examples. However, the present invention is not limited by these examples.

### <Example 1>

### [Detection of Peptide P09 (QLELNER, SEQ ID NO: 19)]

Optimization of a condition for detecting the peptide P09 and detection of the peptide P09 were performed, the peptide P09 having a sequence contained in the VEGF-A variants 206, 189, 183, 165, and 165B and being generated from these variants by trypsin digestion. The peptide P09 was chemically synthesized for the present example.

### [1. Identification of Structure, Confirmation of MS/MS Ion]

First, valence identification of a parent ion P09 to be detected was performed.

The m/z values for calculated valences (+1 - +5) of the peptide P09 are shown in Table 3. Among these, a +2 valence ion was detected in all of LCMS-IT-TOF, LCMS-8040 and LCMS-8050 (Fig. 2-1).

### [2. Optimization of MRM Condition]

Next, an MRM analysis was performed using the +2 valence ion of the peptide P09 as a precursor ion. Fig. 2-2 shows results of an MS2 analysis using LCMS-IT-TOF and analysis results of LCMS-8050.

In a Mascot search, as shown in the lower part of Fig. 2-2, generation of various ions corresponding to six kinds of y ions and six kinds of b ions and various decomposition products of these ions is predicted. However, among these, four kinds of y ions (y(2), y(3), y(4), y(5)) were detected both in an MS2 analysis using LCMS-IT-TOF and in LCMS-8050. Therefore, it was suggested that it is preferable to detect these ions as product ions.

### [3. Optimization of LCMS-8050 MRM Condition]

For each of the selected four kinds of ions (y(2), y(3), y(4), y(5)), an optimum condition of a collision energy in a case of performing detection with MRM using LCMS-8040 and LCMS-8050 was examined. The results are shown in Figs. 2-3 and 2-4.

From the results of Fig. 2-3, it was shown that, in the case where LCMS-8040 was used as an analysis device, the optimum collision energies for detecting the 4 kinds of the ions were all 20 V. Further, from the results of Fig. 2-4, it was shown that, in the case where LCMS-8050 was used as an analysis device, the optimum collision energies for detecting the 4 kinds of the ions were 15 V or 20 V.

### [4. Determination of Column Retention time, MRM Analysis]

Setting of a column retention time was performed using the conditions optimized in 2.3. LCMS-8050 was used as an analysis device. HPLC was performed under the conditions shown in Table 5.

As shown in Fig. 2-5, in a case of detecting the y(2), y(3), y(4) and y(5) ions using a +2 valence ion of the peptide P09 as a precursor ion, a retention time is about 4.6 minutes, and it was found that adequate detection can be performed with a width of 4.20 - 5.20 minutes as a measurement time. A lower part of Fig. 2-5 shows results measured using an established measurement condition.

### <Example 2>

### [Detection of Peptide P14 (SWSVCDKPR, SEQ ID NO: 24)]

Optimization of a condition for detecting the peptide P14 and detection of the peptide P14 were performed, the peptide P14 having a sequence contained only in the VEGF-A variant 145 and being generated from this variant by trypsin digestion. The peptide P14 was chemically synthesized for the present example.

### [1. Identification of Structure, Confirmation of MS/MS Ion]

First, valence identification of a parent ion P14 to be detected was performed.

The m/z values for calculated valences (+1 - +5) of the peptide P14 are shown in Table 3. Among these, a +2 valence ion and a +3 valence ion were detected in all of LCMS-IT-TOF, LCMS-8040 and LCMS-8050 (Fig. 3-1).

### [2. Optimization of MRM Condition (1)]

Next, an MRM analysis was performed using the +2 valence ion of the peptide P14 as a precursor ion. Fig. 3-2 shows results of an MS2 analysis using LCMS-IT-TOF and analysis results of LCMS-8050.

In a Mascot search, as shown in the lower part of Fig. 3-2, generation of various ions corresponding to eight kinds of y ions and eight kinds of b ions and various decomposition products of these ions is predicted. However, among these, one kind of a b ion (b(2)) and six kinds of y ions (y(3), y(4), y(5), y(6), y(7) and y(7)++) were detected both in an MS2 analysis using LCMS-IT-TOF and in LCMS-8050. Further, a y(2) ion was detected with a high peak in LCMS-8050. Therefore, it was suggested that it is preferable to detect these ions as product ions.

### [3. Optimization of LCMS-8050 MRM Condition (1)]

For each of seven kinds of ions (y(2), b(2), y(3), y(7)++, y(5), y(6) and y(7)), an optimum condition of a collision energy in a case of performing detection using LCMS-8050 as an analysis device was examined. The results are shown in Figs. 3-3 and 3-4.

Further, from the results of Figs. 3-3 and 3-4, it was shown that, in the case where LCMS-8050 was used as an analysis device, the optimum collision energies for detecting the above-described ions were in a range of 15 V - 25 V.

### [4. Optimization of MRM Condition (2)]

In parallel with the above sub-section 2, an MRM analysis was performed using the +3 valence ion of the peptide P14 as a precursor ion, and a more suitable analysis condition was examined. Fig. 3-5 shows results of an MS2 analysis using LCMS-IT-TOF and analysis results of LCMS-8050.

Among ions predicted by a Mascot search, four kinds of ions (y(2), y(3), y(4) and y(5)) were detected both in an MS2 analysis using LCMS-IT-TOF and in LCMS-8050. Therefore, it was suggested that it is preferable to detect these ions as product ions.

### [5. Optimization of LCMS-8050 MRM Condition (2)]

For each of the four kinds of the ions (y(2), y(3), y(4) and y(5)), an optimum condition of a collision energy in a case of performing detection using LCMS-8050 as an analysis device was examined. The results are shown in Fig. 3-6.

From the results of Fig. 3-6, it was shown that, in the case where LCMS-8050 was used as an analysis device, the optimum collision energies for detecting the 4 kinds of the ions were 15 V (y(4) and y(5)) or 20 V (y(2) and y(3)).

### [6. Determination of Column Retention time, MRM Analysis]

Setting of a column retention time was performed using the conditions optimized in 4.5. LCMS-8050 was used as an analysis device. HPLC was performed under the conditions shown in Table 5.

As shown in Fig. 3-7, in a case of detecting the y(2), y(3), y(4) and y(5) ions using a +3 valence ion of the peptide P14 as a precursor ion, a retention time is about 4.61 minutes, and it was found that adequate detection can be performed with a width of 4.20 - 5.20 minutes as a measurement time. A lower part of Fig. 3-7 shows results measured using an established measurement condition.

### <Example 3>

In the same way as in Examples 1 and 2, an optimum analysis condition was examined for each of the 10 kinds of the peptides P01 - P16. The results are shown in Fig. 4. Although detailed data is not shown, the results in Fig. 4 were obtained as a result of performing examination for each of the peptides in the same way as that for the peptides P09 and P14.

### <Example 4>

A sample in which the peptides P01 - P16 were mixed at specific quantitative ratios was actually analyzed using the method of the present inventors. More specifically, according to the conditions shown in Fig. 4, an analysis was continuously performed while switching channels at an event time of 0.023 seconds and a dwell time of 20.0 milliseconds. The results are shown in Fig. 5.

From the results of Fig. 5, it is clear that the peptides that are mixed and contained in the sample can be simultaneously detected in parallel. Amounts and relative ratios of the peptides respectively reflected peptide amounts added to the sample, which were measured in advance.

According to the method described herein, conventionally, even when a protein having multiple variants exists, only quantification as a whole or quantification of some of the variants is possible, there was a problem in using such information in a clinical setting where an effect appears as a combined result of mixed variants.

In the method described herein, information on variants can be simultaneously obtained in parallel by an MRM analysis, and the obtained quantitative values can be instantly fed back to a doctor and used as an indicator for clinical medication and treatment options. This provides new medical care that has not been proposed at all in the past.

## Claims

1. A method for parallel determination of multiple splicing variants produced by alternative splicing of a protein having 2 or more variants using mass spectrometry, comprising:
designing peptides obtained by protease digestion of all variants of the protein in a sample;
protease-digesting the protein;
detecting, using mass spectrometry, among the peptides designed, 2 or more kinds of peptides having an amino acid sequence specific to each of the splicing variants and a peptide having an amino acid sequence common to 2 or more splicing variants among the multiple splicing variants; and
determining an amount of each of the splicing variants in the sample based on a result of the mass spectrometry,
wherein the protein is a vascular endothelial growth factor (VEGF)-A.

2. The method according to claim 1, wherein the splicing variants include 2 or more selected from the following splicing variants of VEGF-A: 206 (SEQ ID NO: 1), 189 (SEQ ID NO: 2), 183 (SEQ ID NO: 3), 165 (SEQ ID NO: 4), 148 (SEQ ID NO: 5), 145 (SEQ ID NO: 6), 121 (SEQ ID NO: 7), 165b (SEQ ID NO: 8), 121 b (SEQ ID NO: 9), and 111 (SEQ ID NO: 10).

3. The method according to claim 1 or 2, wherein 1 or more peptides having amino acid sequences shown in SEQ ID NOs: 11 to 26 are detected.

4. A kit for detecting splicing variants of vascular endothelial growth factor (VEGF-A) by executing the method according to any one of claims 1 to 3 using high performance liquid chromatograph mass spectrometry (LC-MS) comprising:
a protease;
a reaction container for digesting the protein by bringing the protein and the protease into contact with each other;
a buffer solution for causing a digestion reaction due to the protease to occur; and
a set of peptides as internal standard peptides having the amino acid sequences shown in SEQ ID NOs: 11 to 26.

5. The kit according to claim 4 further comprising an instruction manual describing a mass spectrometry condition for detecting the splicing variants.

## Patentansprüche

1. Verfahren zur parallelen Bestimmung von multiplen Spleißvarianten, die durch alternatives Spleißen eines Proteins, das 2 oder mehr Varianten aufweist, erzeugt werden, unter Verwendung von Massenspektrometrie, umfassend:
Entwerfen von Peptiden, die durch Proteaseverdau aller Varianten des Proteins in einer Probe erhalten werden;
Verdauen des Proteins mit einer Protease;
Nachweisen, unter Verwendung von Massenspektrometrie, unter den entworfenen Peptiden, von 2 oder mehr Arten von Peptiden, die eine Aminosäuresequenz aufweisen, welche für jede der Spleißvarianten spezifisch ist, und von einem Peptid, das eine Aminosäuresequenz aufweist, welche 2 oder mehr Spleißvarianten unter den multiplen Spleißvarianten gemeinsam ist; und
Bestimmen einer Menge jeder der Spleißvarianten in der Probe, basierend auf einem Ergebnis der Massenspektrometrie,
wobei das Protein ein vaskulärer endothelialer Wachstumsfaktor (VEGF)-A ist.

2. Verfahren nach Anspruch 1, wobei die Spleißvarianten 2 oder mehr beinhalten, ausgewählt aus den folgenden Spleißvarianten von VEGF-A: 206 (SEQ ID NO: 1), 189 (SEQ ID NO: 2), 183 (SEQ ID NO: 3), 165 (SEQ ID NO: 4), 148 (SEQ ID NO: 5), 145 (SEQ ID NO: 6), 121 (SEQ ID NO: 7), 165b (SEQ ID NO: 8), 121b (SEQ ID NO: 9), und 111 (SEQ ID NO: 10).

3. Verfahren nach Anspruch 1 oder 2, wobei 1 oder mehrere Peptide, welche die in SEQ ID NOs: 11 bis 26 gezeigten Aminosäuresequenzen aufweisen, nachgewiesen werden.

4. Kit zum Nachweis von Spleißvarianten des vaskulären endothelialen Wachstumsfaktors (VEGF-A) durch Ausführen des Verfahrens nach einem der Ansprüche 1 bis 3 unter Verwendung von Hochleistungsflüssigkeitschromatographie-Massenspektrometrie (LC-MS), umfassend:
eine Protease;
einen Reaktionsbehälter zum Verdauen des Proteins durch Inkontaktbringen des Proteins und der Protease miteinander;
eine Pufferlösung, um eine Verdauungsreaktion durch die Protease zu bewirken; und
einen Satz von Peptiden als interne Standardpeptide, welche die in SEQ ID NOs: 11 bis 26 gezeigten Aminosäuresequenzen aufweisen.

5. Kit nach Anspruch 4, weiter umfassend eine Gebrauchsanweisung, die eine Massenspektrometriebedingung zum Nachweis der Spleißvarianten beschreibt.

## Revendications

1. Procédé de détermination en parallèle de plusieurs variants d'épissage produits par épissage alternatif d'une protéine ayant 2 variants ou plus, à l'aide de la spectrométrie de masse, comprenant :
la désignation des peptides obtenus par digestion par protéase de tous les variants de la protéine dans un échantillon ;
la digestion par protéase de la protéine ;
la détection, à l'aide de la spectrométrie de masse, parmi les peptides désignés, de 2 types ou plus de peptides ayant une séquence des acides aminés spécifique à chacun des variants d'épissage et d'un peptide ayant une séquence des acides aminés commune aux deux variants d'épissage ou plus parmi les différents variants d'épissage ; et
la détermination de la quantité de chaque variant d'épissage dans l'échantillon sur base du résultat de la spectrométrie de masse,
dans lequel la protéine est un facteur de croissance endothéliale vasculaire (VEGF)-A.

2. Procédé selon la revendication 1, dans lequel les variants d'épissage comprennent 2 variants ou plus choisis parmi les variants d'épissage suivants de VEGF-A : 206 (SEQ ID N°1), 189 (SEQ ID N°2), 183 (SEQ ID N°3), 165 (SEQ ID N°4), 148 (SEQ ID N°5), 145 (SEQ ID N°6), 121 (SEQ ID N°7), 165b (SEQ ID N°8), 121b (SEQ ID N°9), et 111 (SEQ ID N°10).

3. Procédé selon la revendication 1 ou 2, dans lequel 1 ou plusieurs peptides ayant les séquences des acides aminés représentées en SEQ ID N°11 à 26 sont détectés.

4. Kit de détection de variants d'épissage du facteur de croissance endothéliale vasculaire (VEGF-A) par réalisation du procédé selon l'une quelconque des revendications 1 à 3, à l'aide de la chromatographie liquide à haute performance-spectrométrie de masse (LC-MS), comprenant :
une protéase ;
un récipient de réaction pour la digestion de la protéine par mise en contact de la protéine et de la protéase ;
une solution tampon pour permettre à la réaction de digestion induite par la protéase de se produire ; et
un ensemble de peptides en tant que standards internes peptidiques, ayant les séquences des acides aminés représentées en SEQ ID N°11 à 26.

5. Kit selon la revendication 4, comprenant en outre, un manuel d'instructions décrivant les conditions de spectrométrie de masse pour la détection des variants d'épissage.
